(19) Europäisches Patentamt

European Patent Office

Office européen des brevets

(11) **EP 1 146 896 B1**

(12) **EUROPEAN PATENT SPECIFICATION**

(45) Date of publication and mention
of the grant of the patent:
**24.07.2002 Bulletin 2002/30**

(51) Int Cl.⁷: **A61K 38/28**, A61K 47/10,
A61K 47/02, A61K 47/26,
A61P 3/10

(21) Application number: **00904496.7**

(22) Date of filing: **26.01.2000**

(86) International application number:
**PCT/US00/01627**

(87) International publication number:
**WO 00/43034 (27.07.2000 Gazette 2000/30)**

(54) **MONODISPERSE HEXAMERIC ACYLATED INSULIN ANALOG FORMULATIONS**

MONODISPERSE FORMULIERUNGEN, DIE HEXAMERE ACYLIERTE INSULINANALOGE
ENTHALTEN

FORMULATIONS MONODISPERSES D'ANALOGUE ACYLE ET HEXAMERIQUE D'INSULINE

(84) Designated Contracting States:
**AT BE CH CY DE DK ES FI FR GB GR IE IT LI LU
MC NL PT SE**

(30) Priority: **26.01.1999 US 117291 P**

(43) Date of publication of application:
**24.10.2001 Bulletin 2001/43**

(73) Proprietor: **ELI LILLY AND COMPANY
Indianapolis, Indiana 46285 (US)**

(72) Inventors:
• **NG, Kingman
Carmel, IN 46032 (US)**
• **LI, Shun
Indianapolis, IN 46268 (US)**
• **WATTS, Eric, Alan
Greenwood, IN 46142 (US)**

(74) Representative: **Denholm, Anna Marie
Eli Lilly and Company Limited,
Lilly Research Center,
Erl Wood Manor
Windlesham, Surrey GU20 6PH (GB)**

(56) References cited:
**WO-A-97/48414          WO-A-98/34953
WO-A-99/34821**

**Description**

**FIELD OF THE INVENTION**

The invention relates to acylated human insulin analogs. More specifically, the invention relates to pharmaceutical formulations at a pH greater than about 7.9 containing a monoacylated human insulin analog, a metal ion, an isotonicity agent, and a phenolic derivative.

**BACKGROUND OF THE INVENTION**

Since the introduction of therapeutic insulin in the 1920's, continuous efforts have been made to improve the treatment of diabetes mellitus. The availability of insulin replacement therapy has prevented the mortality and morbidity of acute complications in diabetes mellitus. However, chronic diabetic complications remain a major health problem due to persistent metabolic derangement, arising principally from poor control of blood glucose. Results from the Diabetes Control and Complications Trial (DCCT) indicate that a decrease of 1% in HbA1c (glycosylated hemoglobin) correlates with more than 35% improvement in the incidence of retinopathy [The DCCT Research Group, *New Engl. J. Med.*, *329*, 977-986 (1993)].

In order to achieve normal glycemia, therapy must be designed to parallel as closely as possible the pattern of endogenous insulin secretion in normal individuals. The daily physiological demand for insulin fluctuates and can be separated into two phases: (a) the absorptive phase requiring a pulse of insulin to dispose of the meal-related blood glucose surge, and (b) the post-absorptive phase requiring a sustained amount of insulin to regulate hepatic glucose output for maintaining optimal fasting blood glucose. Accordingly, effective therapy involves the combined use of two types of exogenous insulin: a fast-acting meal time insulin and a long-acting basal insulin.

To achieve a long-acting basal action, insulin currently is formulated under conditions favoring formation of a hexamer conformation in an insoluble, crystalline state. These long acting formulations are Ultralente, Lente, and semi-Lente. However, the insolubility of the current long-acting preparations has been shown to cause problems relating to inconsistency in the dose-response as well as unpredictability in time action. In addition, one of the currently available long-acting insulin preparations, beef Ultralente, is immunogenic. The presence of antibodies that result from the immunogenicity of beef Ultralente alters the pharmacokinetics of fast-acting insulins.

While the time action of the insoluble Ultralente formulation makes a convenient once-a-day insulin, many physicians actually prefer to use an intermediate time action insulin, an insulin-protamine formulation commonly referred to as insulin-NPH. Insulin-NPH is used twice daily as a basal insulin because it is comparatively easier to adjust the optimal dosage with a drug of shorter time action. As a result, intermediate-acting insulins account for 70% of the U.S., 64% of the Japanese, 45% of European and an overall 55% of the world-wide insulin market.

However, both insoluble insulin-NPH and insoluble Ultralente insulin are suspension formulations. Thus, the formulations are inherently less predictable than soluble formulations and result in less than adequate control of blood glucose and a greater susceptibility to life-threatening hypoglycemic episodes. One approach to providing basal control of blood glucose has been to acylate insulin with fatty acids and to rely on the binding of fatty acids by serum albumin to retain insulin activity in the circulation for extended periods of time. Thus, considerable research effort has focused on the acylation of human insulin and human insulin analogs.

The acylation of pork, beef, or human insulin is disclosed by Muranishi and Kiso, in Japanese Patent Application 1-254,699. The following compounds are specifically disclosed: B29-N$^\varepsilon$-palmitoyl insulin (the $\varepsilon$-amino group is acylated), B1-N$^\alpha$- palmitoyl insulin (the N terminal $\alpha$-amino group of the B chain is acylated), and B1, B29-N$^\alpha$N$^\varepsilon$-di-palmitoyl insulin (both the $\varepsilon$-amino and the N-terminal $\alpha$-amino group are acylated).

Similarly, Hashimoto, *et al.*, in *Pharmaceutical Research*, *6,* 171-176 (1989), discloses B1- N$^\alpha$-palmitoyl insulin (the N terminal $\alpha$-amino group is acylated), and B1,B29-N$^\alpha$N$^\varepsilon$-dipalmitoyl insulin (both the $\varepsilon$-amino and the N-terminal $\alpha$-amino groups are acylated). Hashimoto, *et al.* studied the hypoglycemic effect of B1-N$^\alpha$- palmitoyl insulin and B1,B29-N$^\alpha$N$^\varepsilon$-dipalmitoyl insulin in male rats at 25 U/mL, an exceedingly high dose. At these doses, Fig. 5 of Hashimoto demonstrates very low activity when administered intravenously. When administered intramuscularly, only a short hypoglycemic effect of B1-N$^\alpha$- palmitoyl insulin and negligible effect of B1,B29-N$^\alpha$N$^\varepsilon$-dipalmitoyl insulin were disclosed in Fig. 6 of Hashimoto.

In addition to the *in vivo* reports by Muranishi and Kiso and Hashimoto *et al.,* Walder *et al.,* in PCT publication WO 92/01476, disclose that the half-life of proteins and peptides can be extended *in vivo* by chemically linking the proteins with an apolar group, specifically a fatty acid derivative. The fatty acid provides a bridging group between the protein and albumin. Walder, *et al.* also disclose that the apolar group is preferably restricted to a unique site or sites in the protein and exemplify the binding of the cysteine residues of hemoglobin. The reference generally discloses fatty acid derivatives of insulin. However, no fatty acid derivatives of insulin are specifically disclosed or exemplified, and no data are disclosed to indicate that the biological activity of the fatty acid derivatives of insulin is retained.

U.S. Patent 5,693,609 issued to Baker *et al.* discloses that selective acylation of a free amino group of a monomeric insulin analog provides an acylated monomeric insulin analog with effective basal insulin activity whereas the unacylated insulin analogs provide a rapid onset of action and a rapid clearance. Specifically disclosed are techniques for acylating Lys$^{B28}$Pro$^{B29}$ human insulin, and the preparation of B28-N$^\varepsilon$-palmitoyl-Lys$^{B28}$Pro$^{B29}$ insulin (the $\varepsilon$-amino group is acylated). However, this reference does not disclose or suggest formulations or compositions containing a monodisperse association of these compounds, nor does it suggest methods for making such formulations or compositions.

It has long been known that natural human insulin forms a hexamer in the presence of zinc (Blundell *et al.*, *Adv. Protein Chem., 26,* 279-402 (1972); and Baker *et al.*, *Philos, Trans. R. Soc. London, B 319*, 369-456 (1988)). The structure of the zinc-insulin hexamer has been characterized thoroughly by a series of X-ray crystal structures (Baker *et al.*, *Philos. Trans. R. Soc. London, B 319,* 369-456 (1988); Derewenda, U., *et al.*, *Nature, 338,* 594-596 (1989); Ciszak, E., *et al.*, *Biochemistry*, *33,* 1512-1517 (1994); Smith, G. D., *et al.*, *Proteins: Struct. Func. Genet., 14,* 401-408 (1992); Smith, G. D., *et al.*, *Biopolymers*, *32,* 1749-1756 (1992)). Other researchers have attempted to increase the stability of hexameric human insulin associations. For example, U.S. Patent No. 4,476,118 issued to Brange *et al.* discloses solutions containing hexamers of bovine, porcine, and human insulin preferably containing between 4.2 and 4.5 $Zn^{2+}$/hexamer. In Examples 1-8, Brange *et al.* discloses that hexamer formation was accomplished in solutions with the pH ranging from 7.4 to 7.5. The quantity of the zinc was found to play a critical role in the stability of the hexameric complex in solution.

Similarly, human insulin palmitoylated at the $\varepsilon$-amino of the Lys at position 29 of the human insulin B chain (N$^\varepsilon$-palmitoyl Lys$^{B29}$ human insulin) has been disclosed to form Zn(II) and Co(II) hexamers under the same conditions used to form hexamers of human insulin ("Effects of Surface Hydrophobicity on the Structural Properties of Insulin", Brader, Mark L. *et al., Techniques in Protein Chemistry VIII: Tenth Symposium of the Protein Society,* pp 289-297, Ed., Marshak, D. R., Academic Press, San Diego, California (1997)).

Finally, U.S. Patent No. 5,474,978 issued to Bakaysa *et al.* discloses that unacylated human insulin analogs form hexamers with zinc and various phenolic compounds at a pH of about 7.5. Specifically disclosed are hexamers of unacylated Lys$^{B28}$Pro$^{B29}$ human insulin. Bakaysa et al. further discloses that the rate of absorption for the hexamer complex is at least two times that observed with insulin. However, when the hexamer complex is formulated, it is reportedly equally stable against chemical degradation.

As discussed above, various references have disclosed the preparation of acylated human insulin analogs. Other references have described the preparation of hexameric associations of human insulin, acylated human insulin and unacylated human insulin analogs. The prior art does not teach or suggest methods for making an acylated insulin analog formulation or composition with the desirable properties of high stability and low precipitation of the acylated insulin analog from solution. In particular, the prior art does not suggest either a pharmaceutical formulation or composition comprising a monodisperse hexameric association of an acylated human insulin analog. Additionally, there are no disclosed or suggested methods for producing such formulations and compositions or treating a patient suffering from hyperglycemia with such formulations and compositions.

Furthermore, practicing the prior art methods of forming hexamers of human insulin, acylated human insulin or unacylated human insulin analogs fails to produce the desired hexamers of acylated human insulin analog. Thus, there is a need for acylated insulin analog formulations and compositions with the desirable properties of high stability and low precipitation of the acylated insulin analog from solution, and there is a need for methods of making such formulations and compositions. As explained above, many people suffer from diabetes, and there are drawbacks with currently-available therapeutic insulin formulations. Therefore, there is a need for improved methods for treating patients suffering from hyperglycemia, using a composition or formulation containing such a composition with the aforementioned desirable properties.

**SUMMARY OF THE INVENTION**

It is therefore an object of the present invention to provide a pharmaceutical formulation, comprising an aqueous solution comprising an isotonicity agent and a monoacylated human insulin analog, or a pharmaceutically acceptable salt thereof, in a monodisperse hexameric association state. The monoacylated human insulin analog comprises the polypeptide of SEQ ID NO:1 properly cross-linked to SEQ ID NO:2, wherein Xaa at position 21 of SEQ ID NO:1 is selected from the group consisting of Asn, Asp, Gly, and Glx; Xaa at position 3 of SEQ ID NO:2 is selected from the group consisting of Asn, Asp, and Glx; Xaa at position 28 of SEQ ID NO:2 is selected from the group consisting of Asp, Leu, Val, Ala, and acylated Lys; Xaa at position 29 of SEQ ID NO:2 is selected from the group consisting of Pro and acylated Lys, and further wherein position 28 or position 29 is acylated Lys, and if position 28 is acylated Lys, position 29 is not acylated Lys, and if position 29 is acylated Lys, position 28 is not acylated Lys. In preferred embodiments of the invention, pharmaceutical formulations include acylated Lys$^{B28}$Pro$^{B29}$ human insulin or acylated Asp$^{B28}$ human insulin in monodisperse hexameric association states.

Another object of the present invention is to provide a pharmaceutical formulation comprising an aqueous solution at a pH of greater than about 7.9 which comprises an isotonicity agent, a phenolic derivative, a metal ion, preferably one or more zinc ions, and a monoacylated human insulin analog, or a pharmaceutically acceptable salt of the analog. The monoacylated human insulin analog comprises the polypeptide of SEQ ID NO:1 properly cross-linked to SEQ ID NO:2, wherein, Xaa at position 21 of SEQ ID NO:1 is selected from the group consisting of Asn, Asp, Gly, and Glx; Xaa at position 3 of SEQ ID NO:2 is selected from the group consisting of Asn, Asp, and Glx; Xaa at position 28 of SEQ ID NO:2 is selected from the group consisting of Asp, Leu, Val, Ala, and acylated Lys; Xaa at position 29 of SEQ ID NO:2 is selected from the group consisting of Pro and acylated Lys, and wherein position 28 or position 29 is acylated Lys, and if position 28 is acylated Lys, position 29 is not acylated Lys, and if position 29 is acylated Lys, position 28 is not acylated Lys.

In a preferred embodiment of the invention, the pH of the formulation is between about 7.9 and 8.0. In another preferred embodiment, the phenolic derivative is selected from the group consisting of phenol, m-cresol and combinations of these, the metal is zinc in the +2 oxidation state, and the formulation includes glycerine and optionally includes a buffer selected from the group consisting of Tris, sodium phosphate, glycylglycine, and citrate. In other preferred embodiments, the monoacylated human insulin analogs are acylated Lys$^{B28}$Pro$^{B29}$ and acylated Asp$^{B28}$ human insulins.

Still another object of the present invention is to provide a method of preparing a pharmaceutical formulation of a monodisperse hexameric association of a monoacylated human insulin analog, including the steps of: contacting a metal in the +2 oxidation state with a monoacylated human insulin analog, or a pharmaceutically acceptable salt of the analog, wherein the monoacylated human insulin analog includes the polypeptide of SEQ ID NO:1 properly cross-linked to SEQ ID NO:2, wherein Xaa at position 21 of SEQ ID NO: 1 is selected from the group consisting of Asn, Asp, Gly, and Glx; Xaa at position 3 of SEQ ID NO:2 is selected from the group consisting of Asn, Asp, and Glx; Xaa at position 28 of SEQ ID NO:2 is selected from the group consisting of Asp, Leu, Val, Ala, and acylated Lys; Xaa at position 29 of SEQ ID NO:2 is selected from the group consisting of Pro and acylated Lys, and wherein position 28 or position 29 is acylated Lys, and if position 28 is acylated Lys, position 29 is not acylated Lys, and if position 29 is acylated Lys, position 28 is not acylated Lys; adding a phenolic derivative and an isotonicity agent, and adjusting the pH of the formulation to greater than about 7.9.

It is another object of the present invention to provide a pharmaceutical formulation, comprising an aqueous solution which comprises an isotonicity agent and a monoacylated human insulin analog, or a pharmaceutically acceptable salt thereof, in a monodisperse hexameric association state. The monoacylated human insulin analog comprises acylated des(B30).

Another object of the present invention is to provide a pharmaceutical formulation comprising an aqueous solution at a pH of greater than about 7.9 including an isotonicity agent, a phenolic derivative, a metal ion, preferably one or more zinc ions, and a monoacylated human insulin analog, or a pharmaceutically acceptable salt of the analog. The monoacylated human insulin analog comprises acylated des(B30) human insulin. In a preferred embodiment of the invention, the pH of the formulation is between about 7.9 and 8.0. In another preferred embodiment, the phenolic derivative is selected from the group consisting of phenol, m-cresol and combinations of these, the metal is zinc in the +2 oxidation state, and the formulation includes glycerine and optionally includes a buffer selected from the group consisting of Tris, sodium phosphate, glycylglycine, and citrate.

Still another object of the present invention is to provide a method of preparing a pharmaceutical formulation of a monodisperse hexameric association of a monoacylated human insulin analog, including the steps of: contacting a metal in the +2 oxidation state with a monoacylated human insulin analog, or a pharmaceutically acceptable salt of the analog, wherein the monoacylated human insulin analog is acylated des(B30) human insulin; adding a phenolic derivative and an isotonicity agent; and adjusting the pH of the metal/analog mixture to greater than about 7.9.

Further objects, features and advantages of the invention will be apparent from the following detailed description taken in conjunction with the accompanying drawings.

**BRIEF DESCRIPTION OF THE DRAWINGS**

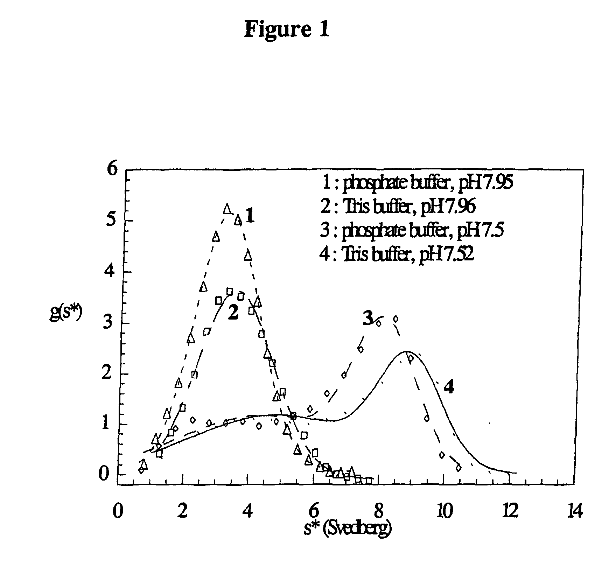

Figure 1 is a graphical representation of the sedimentation coefficient distribution profiles obtained on a Beckman XLI analytical ultracentrifuge for a formulation of B28-N$^\varepsilon$-myristoyl-Lys$^{B28}$Pro$^{B29}$ human insulin analog containing zinc and phenol buffered with 7 mM dibasic sodium phosphate at pH 7.95 (Δ); buffered with 10 mM Tris at pH 7.96 (□); buffered with 7 mM dibasic sodium phosphate at pH 7.5 (◊); and buffered with 10 mM Tris at pH 7.52 (no marker). The graph demonstrates the effect pH has on the association properties of acylated human insulin analogs. The graph also demonstrates formation, in two different buffer systems, of a formulation containing an acylated human insulin analog in a monodisperse hexameric association state.

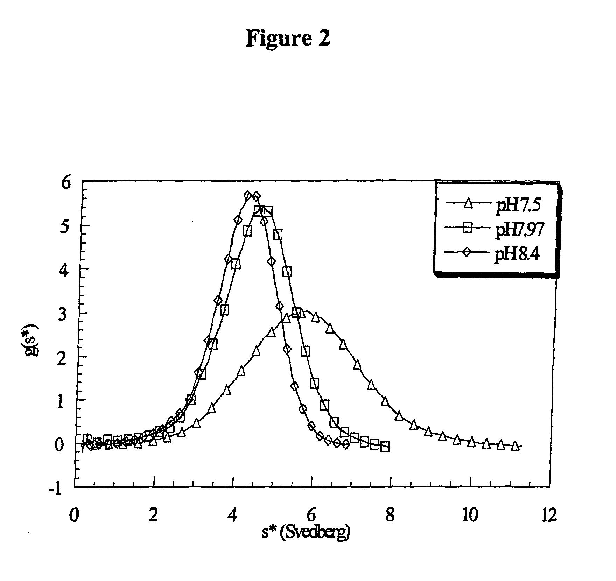

Figure 2 is a graphical representation of the sedimentation coefficient distribution profiles obtained on a Beckman XLI analytical ultracentrifuge for a formulation of B29-N$^\varepsilon$-myristoyl-des(B30) human insulin analog containing zinc and phenol buffered with dibasic sodium phosphate at pH 7.5 (Δ); at pH 7.97 (□); and at pH 8.4 (◊). The graph demonstrates the effect pH has on the association properties of acylated human insulin analogs.

## DETAILED DESCRIPTION AND PREFERRED EMBODIMENTS

All amino acid abbreviations used in this disclosure are those accepted by the United States Patent and Trademark Office as set forth in 37 C.F.R. § 1.822(b)(2).

## Definitions

The term "about," with reference to a pH level, designates a pH range that is plus or minus 0.05 pH units. For example, a pH of "about 8.0" means a pH range of 7.95 to 8.05.

The term "composition" as used herein is a general term used to describe a product of mixing or combining various elements or ingredients. For purposes of this disclosure, the word "composition" includes formulations.

The term "formulation" as used herein refers to a type of composition. The phrase "pharmaceutical formulation" as used herein refers to a type of formulation containing an active compound or salt of an active compound in the presence of other typical pharmaceutical excipients. Typically, pharmaceutical formulations are sterile, particularly those used for parenteral administration.

The term "acylated Lys" refers to Lys acylated at the ε-amino group with a $C_6$-$C_{21}$ fatty acid. The term "acylating group" refers to the fatty acid chemically bonded to the ε-amino group of the insulin analog.

The term "acylating" means covalently linking an acyl group to a free amino group of a protein. An insulin is "acylated" when one or more of its amino groups is combined, through an amide bond, with the acid group of a fatty acid.

The term "human insulin" as used herein refers to human insulin, whose amino acid sequence and spatial structure are well-known. Human insulin is comprised of a twenty-one amino acid A-chain and a thirty amino acid B-chain cross-linked by disulfide bonds between cysteine residues. Human insulin contains three disulfide bridges: one between position 7 of the A-chain and position 7 of the B-chain, a second between position 20 of the A-chain and position 19 of the B-chain, and a third between positions 6 and 11 of the A-chain [Nicol, D. S. H. W. and Smith L. F., *Nature, 187*, 483-485 (1960)].

The term "fatty acid" means a saturated or unsaturated $C_6$-$C_{21}$ fatty acid. The preferred fatty acids for practicing the invention are saturated and include myristic acid (C14), pentadecylic acid (C15), palmitic acid (C16), heptadecylic acid (C17) and stearic acid (C18). Each of these fatty acids is also individually preferred. Also preferred, are fatty acids having between 6 and 10 carbon atoms, between 6 and 9 carbon atoms, and between 6 and 8 carbon atoms. Most preferably, the fatty acid is myristic, palmitic, hexanoic, and octanoic acid . The compositions and formulations of the present invention include monoacylated human insulin analogs. A monoacylated insulin analog comprises one acyl group per insulin analog molecule. In particular, a monoacylated insulin analog of the present invention is acylated with a $C_6$-$C_{21}$ fatty acid at the ε-amino group of one lysine per analog. Preferably, the human insulin analogs of the invention are mono-acylated with a $C_6$-$C_{21}$ fatty acid at the ε-amino group of a lysine at position B28 or B29.

The term "insulin analog" means proteins that have an A-chain and a B-chain that have substantially the same amino acid sequences as the A-chain and B-chain of human insulin, respectively, but differ from the A-chain and B-chain of human insulin by having one or more amino acid deletions, one or more amino acid replacements, and/or one or more amino acid additions that do not destroy the insulin activity of the insulin analog. One type of insulin analog is a "monomeric insulin analog. " Another type of human insulin analog is des(B30) human insulin in which the Thr at position B30 on the B chain of human insulin is absent. Both acylated monomeric insulin analogs and acylated des(B30) human insulin are preferably included in pharmaceutical formulations of the present invention.

The term "monomeric insulin analog" as used herein, designates a fast acting insulin analog that is less prone to dimerization or self-association. A monomeric insulin analog of the invention is human insulin comprising one or more of the following modifications: (1) Pro at position B28 is substituted with Asp, Lys, Leu, Val, or Ala; (2) Lys at position B29 is Lysine or Proline. In addition to these substitutions, the monomeric insulin analog may also comprise one or more of the following modifications: (1) Asn at position A21 may be substituted with Asp, Gly or Glx; (2) Asn at position B3 may be substituted with Asp and Glx. One skilled in the art would recognize that other modifications to the monomeric insulin analog are possible and widely accepted in the art. The monoacylated monomeric human insulin analogs of the compositions and formulations of the present invention include the polypeptide of SEQ ID NO: I properly crosslinked to the polypeptide of SEQ ID NO:2. Other monomeric insulin analogs include des(B27) and des(B28-B30) human insulin.

The term "cross-link" refers to disulfide bridges between cysteine residues. A "properly cross-linked" human insulin, monomeric human insulin analog, human insulin analog, acylated human insulin analog, or acylated monomeric human insulin analog contains three disulfide bridges. One disulfide bridge joins the cysteine residues at position 7 of the A-chain and position 7 of the B-chain. A second disulfide bridge joins the cysteine residues at position 20 of the A-chain and position 19 of the B-chain. The third disulfide bridge joins the cysteine residues at positions 6 and 11 of the A-chain.

SEQ ID NO:1 is a human insulin A-chain, or analog thereof, having the amino acid sequence:

$$1 \quad\quad 5 \quad\quad 10 \quad\quad 15$$

Gly  Ile  Val  Glu  Gln  Cys  Cys  Thr  Ser  Ile  Cys  Ser  Leu  Tyr  Gln

$$20$$

Leu  Glu  Asn  Tyr  Cys  Xaa

wherein:

Xaa at position 21 is Asn, Asp, Gly or Glx.

SEQ ID NO:2 is a human insulin B-chain analog having the amino acid sequence:

$$1 \quad\quad 5 \quad\quad 10 \quad\quad 15$$

Phe  Val  Xaa  Gln  His  Leu  Cys  Gly  Ser  His  Leu  Val  Glu  Ala  Leu

$$20 \quad\quad 25 \quad\quad 30$$

Tyr  Leu  Val  Cys  Gly  Glu  Arg  Gly  Phe  Phe  Tyr  Thr  Xaa  Xaa  Thr

wherein:

Xaa at position 3 is Asn, Asp or Glx;
Xaa at position 28 is Asp, Leu, Val, Ala, or acylated Lys;
Xaa at position 29 is Pro or acylated Lys, and
wherein position 28 or position 29 is acylated Lys, and
if position 28 is acylated Lys, position 29 is not acylated Lys; and
if position 29 is acylated Lys, position 28 is not acylated Lys.

Preferred embodiments of the invention include composition and formulations comprising acylated Lys$^{B28}$Pro$^{B29}$ and acylated Asp $^{B28}$. Other preferred embodiments of the invention include compositions and formulations containing B28-N$^\varepsilon$-palmitoyl-Lys$^{B28}$Pro$^{B29}$, B28-N$^\varepsilon$-myristoyl-Lys$^{B28}$Pro$^{B29}$, B29-N$^\varepsilon$-palmitoyl-Asp $^{B28}$ human insulin, and B29-N$^\varepsilon$-myristoyl-Asp $^{B28}$ human insulin. In other preferred embodiments of the invention, Xaa at position 21 of SEQ ID NO:1 is Asn or Xaa at position 3 of SEQ ID NO:2 is Asn or both Xaa at position 21 of SEQ ID NO: 1 and Xaa at position 3 of SEQ ID NO:2 are Asn.

A "pharmaceutically-acceptable salt" of a monoacylated monomeric human insulin analog or a monoacylated human insulin analog means a salt formed between any one or more of the charged groups in the insulin analog and any one or more pharmaceutically acceptable, non-toxic cations or anions. Organic and inorganic salts include, for example, those prepared from acids such as hydrochloric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, carbonic, and the like. Pharmaceutically-acceptable salts of a mono-acylated monomeric insulin analog and monoacylated human insulin analogs may also contain cations including, but not limited to, ammonium, sodium, potassium, calcium, or magnesium

The term "monodisperse hexameric association" refers to a preparation of an acylated monomeric human insulin analog or an acylated human insulin analog wherein the acylated monomeric human insulin analog or acylated human insulin analog is substantially all in the hexameric association state. Thus, a monodisperse hexameric association of an acylated monomeric human insulin analog refers to a preparation exhibiting a single well-defined peak in its sedimentation coefficient distribution profile corresponding to monoacylated human insulin analog in a hexameric state indicating that 84 percent or greater of the acylated monomeric human insulin analog is in the hexameric association state. More preferably, the sedimentation coefficient distribution profile will indicate that at least 85 percent, at least 90 percent, at least 95 percent, or at least 98 percent of the acylated monomeric human insulin analog is in the hexameric association state. The same peak distribution would be true for a monodisperse hexameric association of a monoacylated human insulin analog.

The term "isotonicity agent" refers to an optional, but highly preferred agent that is physiologically tolerated and imparts a suitable tonicity to the formulation to prevent the net flow of water across the cell membrane. Compounds including, but not limited to, glycerine, and various carbohydrates such as sucrose, dextrose, mannitol, and trehalose or mixtures thereof, are commonly used for such purposes at known concentrations. Thus, each of these compounds, individually or in mixtures, is a preferred isotonicity agent. For example, glycerine, also referred to as glycerol, at a concentration of about 12 to about 25 mg/mL is a preferred isotonicity agent in the present invention. Most preferably, glycerine is present at a concentration of about 16 mg/mL. Thus, glycerine is preferably present at an amount ranging from about 1.2 to about 2.5 percent by weight, but is more preferably present in an amount of about 1.6 or 1.5 percent by weight. Carbohydrates such as mannitol, a preferred isotonicity agent, are preferably used in amounts ranging from about 10 to about 60 mg/mL and are more preferably used in amounts ranging from about 20 to about 40 mg/mL. Even more preferably, carbohydrates are used in an amount of about 30 to about 38 mg/mL, and most preferably they are used in an amount of about 36 mg/mL. Although glycerine or a carbohydrate such as those listed above may be individually used as an isotonicity agent, combinations of these isotonicity agents may be used in certain preferred pharmaceutical formulations of the present invention. Sodium chloride may also be used as an isotonicity agent by itself, but preferably is combined with other isotonicity agents such as glycerine or mannitol. Preferably, if sodium chloride is present in a pharmaceutical formulation of the present invention, it is present in an amount of less than 150 mM.

The term "phenolic derivative" refers to phenol, m-cresol, p-cresol, o-cresol, chloro-cresol, benzyl alcohol, methylparaben, resorcinol, phenylethanol, phenoxyethanol, and mixtures thereof. The most preferred phenolic derivatives of the present invention include phenol and m-cresol.

The term "preservative" refers to a compound that is added to compositions and pharmaceutical formulations to act as an anti-microbial agent. One type of preservative is a phenolic derivative. Other preservatives known as being effective in pharmaceutical formulations include benzalkonium chloride, benzethonium, chlorohexidine, phenol, m-cresol, benzyl alcohol, methylparaben, chlorobutanol, o-cresol, p-cresol, chlorocresol, resorcinol, phenylmercuric nitrate, thimerosal, benzoic acid, and various mixtures thereof. A particularly preferred mixture of phenols for use in the present invention includes a mixture of m-cresol and phenol.

The terms "buffer" and "physiologically tolerated buffer" are known in the art. A physiologically tolerated buffer is preferably Tris, a phosphate buffer like sodium phosphate, or arginine. Tris is a name commonly used by those skilled in the art for tris(hydroxymethyl)aminomethane. Other physiologically tolerated buffers include, but are not limited to, sodium acetate, sodium citrate, glutamate and carbonate, and glycylglycine. Each of these buffers is preferred for use in the formulations of the present invention. Especially preferred buffers include Tris, sodium phosphate, glycylglycine, and citrate. Additionally, it is possible and preferable to use mixtures of these buffers in the formulations of the present invention.

The term "treating" as used herein, describes the management and care of a patient for the purpose of combating a disease, condition, or disorder and includes the administration of a formulation of the present invention to prevent the onset of the symptoms or complications, to alleviate the symptoms or complications, or to eliminate the disease, condition, or disorder.

The term "effective amount" refers to that amount of one or more acylated analogs of the present invention needed to lower or maintain blood sugar levels, either therapeutically or prophylactically. This amount typically may range upward from about 10 units or more per day (or about 0.3 to about 2 mg assuming approximately 27.5 units per mg for human insulin). However, it is to be understood that the amount of the acylated analog(s) actually administered will be determined by a physician in light of the relevant circumstances including: the condition being treated, the cause of the hyperglycemia, the particular insulin analog to be administered, the chosen route of administration, the age, weight and responsiveness of the individual patient and the severity of the patient's symptoms. Thus, the above dosage range is not intended to limit the scope of the invention in any manner. It is well within the skill of the art to adjust the dosage range to fit each patient's particular needs.

One or more formulations of the present invention is administrable to a patient in need thereof -- a patient suffering from hyperglycemia. An effective amount of a monodisperse hexameric association formulation of a monoacylated monomeric human insulin analog or a monoacylated human insulin analog of the invention is administrable to a patient suffering from hyperglycemia or diabetes. The pharmaceutical formulations of the present invention may typically be formulated so as to contain about 100 units per mL, or similar concentrations containing an effective amount of the monodisperse hexameric association of monoacylated monomeric human insulin analog or monoacylated human insulin analog. Accordingly, the present invention includes acylated insulin analog formulations in unit dosage form, such as a vial or ampoule containing 100 units per mL. As noted above, a typical unit dose of an acylated insulin analog of the present invention is about 10 units or more per day. These formulations are typically, though not necessarily, parenteral in nature and may be prepared by a variety of techniques which are known by those skilled in the art.

However, the compositions may also be prepared for pulmonary or other delivery methods known to those skilled in the art.

For example, the inventive pharmaceutical formulations can be delivered using any of a variety of devices and methods suitable for administration by inhalation described here or known in the art, for example using a nebulizer or a sprayer. Such devices require the use of formulations suitable for the administration for the dispensing the acylated human insuiln analog formulations in an aerosol. Such aerosols can comprise a solution. Nebulizers like AERx™ Aradigm, the Ultravent® nebulizer (Mallinckrodt), and the Acorn II® nebulizer (Marquest Medical Products) (US 5404871 Aradigm, WO 97/22376), produce aerosols from solutions. These specific examples of commercially available inhalation devices are intended to be representative of specific devices suitable for the practice of this invention, and are not intended as limiting the scope of the invention.

A spray including an insulin formulation of the invention can be produced by forcing the formulation from a nozzle under pressure. The inventive insulin formulations of the invention also can be administered by a nebulizer, such as jet nebulizer or an ultrasonic nebulizer. The insulin formulations of the invention that are administrable via a pulmonary route can include one or more of the following components: an isotonicity agent, a metal ion, a phenolic derivative, a buffer, an excipient, a surfactant, and a bulk protein.

## Preparation of Insulin Analogs

The monoacylated insulin analogs of the present invention are prepared by first synthesizing the insulin analog portion of the molecule, and then acylating the insulin analog.

The insulin analogs of the present invention are synthesized by well-known chemical methods alone, by recombinant DNA methods alone, or by a combination of chemical and recombinant DNA methods. The following list briefly outlines several basic methods for synthesizing these insulin analogs. The list is not exhaustive, and variations of the basic schemes are possible.

1) Synthesis of a human insulin A-chain analog (SEQ ID NO: 1) by standard biosynthetic methods using recombinant DNA technology and separate synthesis of a human insulin B-chain or a human insulin B-chain analog (SEQ ID NO:2) by standard biosynthetic methods using recombinant DNA technology, followed by cross-linking the chains using known disulfide chemistry, as disclosed in Chance, *et al*., U.S. Patent 4,421,685, which is incorporated expressly herein by reference.

2) Synthesis of a human insulin A-chain analog (SEQ ID NO:1) by standard chemical synthesis methods and separate synthesis of a human insulin B-chain or a human insulin B-chain analog (SEQ ID NO:2) by standard chemical synthesis methods, followed by their cross-linking via known disulfide chemistry, as referred to above.

3) Chemical synthesis of one of the chains and biosynthesis using recombinant DNA technology, of the other chain, followed by their cross-linking via known disulfide chemistry, as referred to above.

4) Cleavage of a proinsulin-like precursor, which is made by chemical synthesis, or, preferably, by biosynthesis using recombinant DNA technology, using an enzyme having trypsin-like activity.

### *Chemical Synthesis*

The principles of solid phase chemical synthesis of polypeptides are well-known and may be found in general texts in the area. [*See*, *e.g*., H. Dugas and C. Penney, BIOORGANIC CHEMISTRY, Springer-Verlag, New York, pp. 54-92 (1981).] Such methods can be used for the aforementioned chemical synthesis of insulin and insulin analogs of the invention. Thus, polypeptides of the present invention may be synthesized by solid-phase methodology utilizing an Applied Biosystems 430A peptide synthesizer (commercially available from Applied Biosystems, Foster City California) and synthesis cycles supplied by Applied Biosystems. Protected amino acids, such as *t*-butoxycarbonyl-protected amino acids, and other reagents used in such chemical syntheses, are commercially available from many chemical supply houses.

Sequential *t*-butoxycarbonyl chemistry, using double couple protocols, is applied to the starting *p*-methyl benzhydryl amine resins for the production of C-terminal carboxamides. For the production of C-terminal acids, the corresponding pyridine-2-aldoxime methiodide resin is used. Asparagine, glutamine, and arginine are coupled using preformed hydroxybenzotriazole esters. The following side chain protection may be used:

Arg, Tosyl
Asp, cyclohexyl
Glu, cyclohexyl
Ser, Benzyl
Thr, Benzyl

Tyr, 4-bromo carbobenzoxy

Removal of the *t*-butoxycarbonyl moiety (deprotection) may be accomplished with trifluoroacetic acid (TFA) in methylene chloride. Following completion of the synthesis, the peptides may be deprotected and cleaved from the resin with anhydrous hydrogen fluoride containing 10% meta-cresol. Cleavage of the side chain protecting group(s) and of the peptide from the resin is carried out at zero degrees centigrade or below, preferably -20°C for thirty minutes followed by thirty minutes at 0°C.

After removal of the hydrogen fluoride, the peptide/resin is washed with ether, and the peptide extracted with glacial acetic acid and then lyophilized. Purification is accomplished by size-exclusion chromatography, such as, on a Sephadex G-10 (Pharmacia) column in 10% acetic acid.

### Synthesis using Recombinant DNA Technology

The individual A- and B-chains of the present invention or a proinsulin-like precursor of the insulin analogs of the present invention may be biosynthesized starting from recombinant DNA. Recombinant methods are preferred if a high yield is desired. A general method for the construction of any desired DNA sequence is provided in J. Brown, *et al.*, *Methods in Enzymology, 68*,109 (1979). *[See also,* J. Sambrook, *et al.,* MOLECULAR CLONING: A LABORATORY MANUAL, Cold Spring Harbor, New York (1989)].

The basic steps in the recombinant production of desired proteins are:

a) constructing a synthetic or semi-synthetic DNA encoding the protein of interest;
b) integrating the DNA into an expression vector in a manner suitable for the expression of the protein of interest, either alone or as a fusion protein;
c) transforming an appropriate eukaryotic or prokaryotic host cell with the expression vector,
d) culturing the transformed or transfected host cell in a manner to express the protein of interest; and
e) recovering and purifying the recombinantly produced protein of interest.

In general, prokaryotes are used for cloning of DNA sequences in constructing the vectors of this invention. Prokaryotes may also be employed in the production of the protein of interest. For example, the *Escherichia coli* K12 strain 294 (ATCC No. 31446) is particularly useful for the prokaryotic expression of foreign proteins. Other strains of *E. coli* which may be used (and their relevant genotypes) include the following.

Table 1.

| Genotypes of *E. coli* that may be used to synthesize insulin analogs. | |
|---|---|
| **Strain** | **Genotype** |
| DH5α | F- (φ80dlacZΔM15), Δ(lacZYA-argF)U169 supE44, λ-, hsdR17($r_K^-$, $m_K^+$), recA1, endA1, gyrA96, thi-1, relA1 |
| HB101 | SupE44, hsdS20($r_B^-$ $m_B^-$), recA13, ara-14, proA₂ lacY1, galK2, rpsL20, xyl-5, mtl-1, mcrB, mrr |
| JM109 | RecA1, e14-(mcrA), supE44, endA1, hsdR17($r_K^-$, $m_K^+$), gyrA96, relAl, thi-1, (lac-proAB), F'[traD36, proAB + lacl$_q$,lacZ M15] |
| RR1 | SupE44, hsdS20($r_B^-$ $m_B^-$), ara-14 proA₂, lacY1, galK2, rpsL20, xyl-5, mtl-5 |
| χ1776 | F-, ton, A53, dapD8, minA1, supE42 (glnV42), Δ(gal-uvrB)40, minB2, rfb-2, gyrA₂5, thyA142, oms-2, metC65, oms-1, Δ(bioH-asd)29, cycB2, cycA1, hsdR2, λ- |
| 294 | EndA, thi-, hsr-, hsm$_K^+$ (U.S. Patent 4,366,246) |
| LE392 | F-, hsdR514 (r⁻m⁻), supE44, supF58, lacY1, or Δlac(I-Y)6, galK2, glaT22, metB1, trpR55, λ- |

These strains are all commercially available from suppliers such as: Bethesda Research Laboratories, Gaithersburg, Maryland 20877 and Stratagene Cloning Systems, La Jolla, California 92037; or are readily available to the public from sources such as the American Type Culture Collection, 12301 Parklawn Drive, Rockville, Maryland, 10852-1776.

Except where otherwise noted, these bacterial strains can be used interchangeably. The genotypes listed are illustrative of many of the desired characteristics for choosing a bacterial host and are not meant to limit the invention in any way. The genotype designations are in accordance with standard nomenclature. *[See, for example*, J. Sambrook, *et al*., *supra.]* A preferred strain of *E. coli* employed in the cloning and expression of the genes of this invention is RV308, which is available from the ATCC under accession number ATCC 31608, and is described in United States Patent 4,551,433, issued November 5, 1985.

In addition to the strains of *E. coli* discussed *supra,* bacilli such as *Bacillus subtilis*, other enterobacteriaceae such as *Salmonella typhimurium* or *Serratia marcescans,* and various *Pseudomonas* species may be used. In addition to these gram-negative bacteria, other bacteria, especially *Streptomyces,* spp., may be employed in the prokaryotic cloning and expression of the proteins of this invention.

Promoters suitable for use with prokaryotic hosts include the β-lactamase [vector pGX2907 (ATCC 39344) contains the replicon and β-lactamase gene] and lactose promoter systems [Chang, *et al*., *Nature (London), 275,* 615 (1978); and Goeddel, *et al*., *Nature (London)*, *281,* 544 (1979)], alkaline phosphatase, the tryptophan (trp) promoter system [vector pATH1 (ATCC 37695) is designed to facilitate expression of an open reading frame as a trpE fusion protein under control of the trp promoter] and hybrid promoters such as the tac promoter (isolatable from plasmid pDR540 ATCC-37282). However, other functional bacterial promoters, whose nucleotide sequences are generally known, enable one of skill in the art to ligate them to DNA encoding the proteins of the instant invention using linkers or adapters to supply any required restriction sites. Promoters for use in bacterial systems also will contain a Shine-Dalgarno sequence operably linked to the DNA encoding the desired polypeptides. These examples are illustrative rather than limiting.

The proteins of this invention may be synthesized either by direct expression or as a fusion protein comprising the protein of interest as a translational fusion with another protein or peptide which may be removable by enzymatic or chemical cleavage. It is often observed in the production of certain peptides in recombinant systems that expression as a fusion protein prolongs the lifespan, increases the yield of the desired peptide, or provides a convenient means of purifying the protein of interest. A variety of peptidases (e.g., trypsin) which cleave a polypeptide at specific sites or digest the peptides from the amino or carboxy termini (e.g. dipeptidylaminopeptidase) of the peptide chain are known. Furthermore, particular chemicals (e.g. cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. [*See*, *e.g.,* P. Carter, "Site Specific Proteolysis of Fusion Proteins", Chapter 13 in PROTEIN PURIFICATION: FROM MOLECULAR MECHANISMS TO LARGE SCALE PROCESSES, American Chemical Society, Washington, D.C. (1990)].

### *Acylation*

The acylation of free amino groups of proteins, including insulin, is known in the art. General methods of acylation are set forth in *Methods of Enzymology*, *25,* 494-499 (1972) and include the use of activated esters, acid halides, or acid anhydrides. The use of activated esters, in particular N-hydroxysuccinimide esters, of fatty acids is a particularly advantageous means of acylating a free amino acid with a fatty acid. Lapidot *et al*., *J. of Lipid Res. 8,* 142-145 (1967). Lapidot *et al.* describe the preparation of N-hydroxysuccinimide esters and their use in the preparation of N-lauroyl-glycine, N-lauroyl-L-serine, and N-lauroyl-L-glutamic acid.

To selectively acylate the ε-amino group, various protecting groups may be used to block the α-amino groups during the coupling. The selection of a suitable protecting group is known to one skilled in the art and includes *p*-methoxybenzoxy-carbonyl (pmZ). Preferably, the ε-amino group is acylated in a one step synthesis without the use of amino-protecting groups. The acylation is carried out by reacting the activated fatty acid ester with the ε-amino group of the protein under basic conditions in a polar solvent. The basicity of the reaction must be sufficient to deprotonate all the free amino groups of the insulin analog. Under weakly basic conditions, all the free amino groups are not deprotonated and preferential acylation of the N-terminal or α-amino groups results. In an aqueous solvent or co-solvent, basic conditions means the reaction is carried out at a pH greater than 9.0. Because protein degradation results at a pH range exceeding 12.0, the pH of the reaction mixture is preferably 10.0 to 11.5, and most preferably 10.5. The pH measurement of the reaction of the reaction mixture in a mixed organic and aqueous solvent is the pH of the aqueous solvent prior to mixing.

In a non-aqueous solvent, the selective acylation of the ε-amino group is carried out in the presence of a base with basicity equivalent to a $pK_a$ greater than or equal to 10.75 in water in order to sufficiently deprotonate the ε-amino group. That is, the base must be at least as strong as triethylamine. Preferably, the base is tetramethylguanidine, diisopropylethylamine, or tetrabutylammonium hydroxide. The use of a weaker base results in the acylation of the α-amino groups.

The choice of solvent is not critical and dependent largely on the solubility of the insulin analog and the fatty acid ester. The solvent may be wholly organic. Generally acceptable organic solvents include DMSO, DMF and the like. Aqueous solvent and mixtures of aqueous and organic solvents are also operable. The selection of the polar solvents is limited only by the solubility of the reagents. Preferred solvents are DMSO; DMF; acetonitrile and water; acetone and water; ethanol and water; isopropyl alcohol and water; isopropyl alcohol, ethanol, and water; and ethanol, propanol and water. Preferably, the solvent is acetonitrile and water; most preferably 50 % acetonitrile. One skilled in the art would recognize that other polar solvents are also operable.

Generally, it is preferred that the activated fatty acid ester be in molar excess. Preferably the reaction is carried out with 1 to 4 molar equivalents, most preferably 1 to 2 molar equivalents, of the ester. One skilled in the art would recognize that at very high levels of activated ester, di- or tri-acylated product will be produced in significant quantity.

The temperature of the reaction is not critical. The reaction is carried out at between 20 to 40 degrees Celsius and is generally complete in 15 minutes to 24 hours.

After acylation, the product is purified by standard methods such as reverse phase or hydrophobic chromatography. Thereafter, the product is recovered by standard methods such as freeze drying or by crystallization.

**Preparation of Inventive Compositions and Formulations**

It has been discovered that monoacylated monomeric human insulin analogs and monoacylated human insulin analogs are converted to compositions containing a monodisperse hexameric association by contacting a metal in the +2 oxidation state with a monoacylated human insulin analog, or a pharmaceutically acceptable salt of the analog, adding a phenolic derivative and optionally an isotonicity agent to the mixture, and adjusting the pH to greater than about 7.9.

Earlier attempts to prepare compositions and formulations containing monodisperse hexameric associations of acylated monomeric human insulin analogs and acylated human insulin analogs failed. In these early attempts, solutions of the acylated monomeric human insulin analog were prepared by dissolving the acylated insulin analog in acidic aqueous solutions at a pH of 2.5 to give a concentration of about 3.6 mg/mL. Next, zinc oxide in an amount ranging from 2.0 to 2.5 moles per 6 moles of acylated human insulin analog was added. The pH of the resulting solution was adjusted to about 7.5 by adding a base such as sodium hydroxide. Next, aliquots ranging from 1.0 to 2.0 mL were removed and added to vials and then lyophilized to produce a powder. A diluent solution at a pH of about 7.5 and comprising 16 mg/mL of an isotonicity agent such as glycerine, 5 mg/mL of a phenolic derivative such as phenol, and a buffer such as Tris (10 mM) or dibasic sodium phosphate heptahydrate (7 mM), was then added to vials containing the lyophilized powder. Sedimentation velocity experiments were performed, and the results indicated that the solutions were heterogeneous with respect to association. In other words, there was no monodisperse hexameric association composition present. Thus, standard methods failed to produce the monodisperse hexameric association compositions and formulations, and the results indicated it was not possible to form monodisperse hexameric associations of the acylated human insulin analogs. Surprisingly and unexpectedly, it was discovered that a shift in the pH produced dramatically different results and provided the desired monodisperse hexameric association compositions and formulations.

It has been discovered that at a pH of greater than about 7.9, preparation of compositions and formulations containing monodisperse hexameric associations of the acylated monomeric human insulin analogs and the acylated human insulin analogs can be accomplished. One preferred pH range for the compositions and formulations of the invention is between about 7.9 and about 8.3. A more preferred pH range is between about 7.9 and 8.0. Another preferred pH range is between about 7.9 and about 8.4.

A solution containing metal ions and monoacylated monomeric human insulin analog or monoacylated human insulin analog is prepared by dissolving the analog in an aqueous acidic solution and then adding 2 to 2.5 moles of metal ion per 6 moles of monoacylated monomeric human insulin analog. In another preferred embodiment, 2.5 to 3 moles of metal ion is added per 6 moles of monoacylated human insulin analog. In yet another preferred embodiment, 2-3 moles of metal ion is added per 6 moles of monoacylated human insulin analog. Preferably, the pH of the aqueous solution used to dissolve the analog ranges from about 2.0 to 3.0. More preferably, the pH of the solution used to dissolve the analog is about 2.5.

A metal ion, preferably in the +2 oxidation state, and preferably zinc or cobalt, and most preferably zinc, is added to the dissolved monoacylated monomeric human insulin analog or monoacylated human insulin analog in the form of various metal species. For example, zinc can be added in the form of zinc oxide, zinc nitrate, zinc phosphate, zinc acetate, zinc chloride, zinc bromide, zinc iodide, zinc fluoride, and zinc sulfate. Those skilled in the art will recognize that there are many alternative metal salts that might be used in the process of the present invention. The particular salt form of the metal species is not of particular relevance, however, the salt that is used should be pharmaceutically acceptable. Preferably, zinc is added in the form of zinc oxide. Once the addition of the metal is complete, the resulting solution is generally adjusted to a pH of about 7.5 by addition of a base such as, but not limited to, sodium hydroxide. Those skilled in the art will recognize that this initial solution can have various other pHs without adversely influencing the later formation of the monodisperse hexamer association of the monoacylated monomeric human insulin analogs or the monoacylated human insulin analogs.

Although removal of solvent is not required, preferably solutions containing a metal ion and a monoacylated monomeric human insulin analog or a monoacylated human insulin analog are lyophilized to produce powders that may be reconstituted with appropriate diluent solutions. The term "diluent solution" describes a solution that is added to a lyophilized powder to dissolve the lyophilized powder. The process of dissolving the lyophilized powder with the diluent is referred to as "reconstitution".

Diluent solutions generally contain a phenolic derivative, although various other materials such as, but not limited to, isotonicity agents may be present. Preferably, the diluent solution will contain a phenolic derivative, a buffer, and an isotonicity agent. More preferably, the diluent solution will contain glycerine, a phenolic derivative, and a buffer selected from the group consisting of Tris, dibasic sodium phosphate, glycylglycine, and citrate. The pH of the diluent solution can range considerably, and it is not necessary that the pH of the diluent solution be equal to that of the final composition or formulation because a base such as sodium hydroxide, potassium hydroxide, or others known to those skilled in the art can be used to adjust the pH upward and acids such as hydrochloric, hydrobromic, and others known to those skilled in the art can be added to adjust the pH downward.

Once the lyophilized powder has been reconstituted with a diluent solution, the pH of the solution is adjusted by addition of base or acid, as described above, to attain a pH of greater than about 7.9. This final adjustment to pH will not be necessary if the pH of the solution is already in the range sufficient to produce a composition or formulation containing the monodisperse hexameric association of the monoacylated monomeric human insulin analog or the monoacylated human insulin analog.

It will be recognized by one skilled in the art that the compositions and formulations of the present invention may be prepared by alternative routes. For example, the metal ion might be added to an aqueous acidic solution followed by addition and dissolution of the monoacylated monomeric human insulin analog or the monoacylated human insulin analog. The phenolic derivative might then be added to the sample and the pH adjusted to give a final value greater than about 7.9. Furthermore, one skilled in the art will recognize that the concentrations of the various ingredients can be modified and that additional materials may be added to the compositions and formulations of the present invention.

The compositions and pharmaceutical formulations of the present invention include an acylated monomeric human insulin analog or an acylated human insulin analog in a monodisperse hexameric association state. The acylated monomeric human insulin analog includes a polypeptide having the sequence of SEQ ID NO: 1 properly cross-linked to a polypeptide having the sequence of SEQ ID NO:2. Other acylated human insulin analogs such as acylated des (B30) human insulin are particularly useful and employed in the pharmaceutical formulations of the present invention.

The compositions and pharmaceutical formulations of the present invention further comprise a phenolic derivative, and a metal ion, and have a pH of greater than about 7.9. Preferably, the pH of the composition or formulation ranges between about 7.9 and 8.3, and most preferably the pH ranges between about 7.9 and 8.0. Additionally, formulations with pH values of about 8.4 have also been found useful to prepare monodisperse hexameric associations of acylated monomeric human insulin analogs and acylated human insulin analogs. Thus, pH ranges of greater than about 7.9 and less than or about and including 8.4 are also preferred. The metal ion in the compositions and pharmaceutical formulations is preferably in the +2 oxidation state and is preferably cobalt or zinc. Most preferably, the metal ion is zinc in the +2 oxidation state.

As discussed above, the compositions and pharmaceutical formulations of the present invention may contain other ingredients that include, but are not limited to, buffers, other salts, and isotonicity agents such as carbohydrates. Preferred monoacylated monomeric human insulin analogs and monoacylated human insulin analogs of the invention include B28-N$^\varepsilon$-palmitoyl-Lys$^{B28}$Pro$^{B29}$, B28-N$^\varepsilon$-myristoyl-Lys$^{B28}$Pro$^{B29}$, B28-N$^\varepsilon$-hexanoyl-Lys$^{B28}$Pro$^{B29}$, B28-N$^\varepsilon$-octanoyl-Lys$^{B28}$Pro$^{B29}$, B29-N$^\varepsilon$-palmitoyl-Asp$^{B28}$ human insulin, B29-N$^\varepsilon$-myristoyl-Asp$^{B28}$ human insulin B29-N$^\varepsilon$-hexanoyl-Asp$^{B28}$ human insulin, B29-N$^\varepsilon$-octanoyl-Asp$^{B28}$ human insulin, B29-N$^\varepsilon$-myristoyl-des(B30) human insulin, B29-N$^\varepsilon$-palmitoyl-des(B30) human insulin, B29-N$^\varepsilon$-octanoyl-des(B30) human insulin, and B29-N$^\varepsilon$-hexanoyl-des(B30) human insulin.

The pharmaceutical formulations of the present invention are preferably in a solution state. More preferably, they are an aqueous solution that is essentially or substantially free of organic solvents, particularly methanol, ethanol, propanol, butanol, pentanol, i-propanol, and other similar primary and secondary alcohols. Thus, preferred formulations preferably contain less than 2 percent or more preferably less than 1 percent of these alcohols. The formulations are preferably substantially to completely free of other solvents which might result in haziness or would result in precipitation of the monoacylated human insulin analog or the monoacylated monomeric human insulin analog. The formulations of the present invention are preferably free of precipitated materials and are more preferably free of precipitated monoacylated human insulin analog or monoacylated monomeric human insulin analog. Thus, the more preferred compositions and formulations have less than two percent or more preferably less than 1 percent of the monoacylated human insulin analog or monoacylated monomeric human insulin analog precipitated out of solution, and the most preferred compositions and formulations have all the protein in solution. Thus, preferred compositions and formulations according to the present invention are also free of any material that would result in precipitation of the acylated insulin analogs. Preferred compositions and formulations are clear solutions that most preferably are not hazy in appearance. The addition of some organic solvents such as those primary and secondary alcohols enumerated above may result in hazy solutions.

It will be recognized by one skilled in the art that the compositions and formulations of the present invention may contain additional ingredients. For example, various salts such as sodium or potassium chloride may be added to the compositions and formulations without deleterious effect. As noted above, sodium chloride can be used as an isotonicity agent when it is present at a concentration of less than 150 mM, in a pharmaceutical formulation of the present invention.

The following examples and preparations are provided merely to further illustrate the preparation of compositions and formulations of monodisperse hexameric associations of acylated monomeric human insulin analogs or acylated human insulin analogs of the invention. The scope of the invention is not to be construed as merely consisting of the following examples.

## PREPARATION AND ANALYSIS OF EXAMPLES

## EXAMPLE 1

### Preparation of Lyophilized Zn/B28-N$^{\varepsilon}$-Myristoyl-Lys$^{B28}$Pro$^{B29}$ Insulin Analog Sample Vials

Lyophilized samples of B28-N$^{\varepsilon}$-myristoyl-Lys$^{B28}$Pro$^{B29}$ were prepared by dissolving B28-N$^{\varepsilon}$-myristoyl-Lys$^{B28}$Pro$^{B29}$ in sterile water at a pH of about 2.5 to achieve a concentration of about 3.6 mg/mL. Zinc oxide was then added to the dissolved acylated monomeric insulin analog in an amount ranging from about 2 to 2.5 moles of zinc oxide per 6 moles of monoacylated monomeric insulin analog. After mixing, the pH of the solution was adjusted to 7.5 by addition of sodium hydroxide. Aliquots of 2.0 mL were then injected into vials and lyophilized for future use.

## EXAMPLE 2

### Preparation of B28-N$^{\varepsilon}$-Myristoyl-Lys$^{B28}$Pro$^{B29}$ Insulin Analog Formulations with Tris Buffer at Different pH's

To a vial containing a lyophilized powder of 6.8 mg B28-N$^{\varepsilon}$-myristoyl-Lys$^{B28}$Pro$^{B29}$ and 2 moles of Zn$^{2+}$ per 6 moles of monoacylated monomeric human insulin analog, was added 1.9 mL of a 10 mM aqueous diluent solution of Tris containing 5 mg/mL phenol and 16 mg/mL of glycerine. After reconstitution, the pH was checked and determined to be 7.58. The pH was then downwardly adjusted to 7.52 by addition of hydrochloric acid. A small quantity (0.5 mL) of the reconstituted material was saved and the rest of the solution was placed in a second vial, and sodium hydroxide was added until a pH of 7.96 was achieved.

## EXAMPLE 3

### Preparation of B28-N$^{\varepsilon}$-Myristoyl-Lys$^{B28}$Pro$^{B29}$ Insulin Analog Formulation at a pH of 7.5 with Phosphate Buffer

To a vial containing a lyophilized powder of 6.8 mg B28-N$^{\varepsilon}$-myristoyl-Lys$^{B28}$Pro$^{B29}$ and 2 moles of Zn$^{2+}$ per 6 moles of monoacylated monomeric human insulin analog, was added 1.9 mL of a 7 mM aqueous diluent solution of dibasic sodium phosphate heptahydrate containing 5 mg/mL phenol and 16 mg/mL of glycerine. After reconstitution, the pH of the mixture was checked and determined to be 7.5.

## EXAMPLE 4

### Preparation of B28-N$^{\varepsilon}$-Myristoyl-Lys$^{B28}$Pro$^{B29}$ Insulin Analog Formulation at a pH of 7.95 with Phosphate Buffer

To a vial containing a lyophilized powder of 6.8 mg B28-N$^{\varepsilon}$-myristoyl-Lys$^{B28}$Pro$^{B29}$ and 2 moles of Zn$^{2+}$ per 6 moles of monoacylated monomeric human insulin analog, was added 1.9 mL of a 7 mM aqueous diluent solution of dibasic sodium phosphate heptahydrate containing 5 mg/mL phenol and 16 mg/mL of glycerine. After reconstitution, the pH of the mixture was checked and determined to be 7.85. Sodium hydroxide was added to bring the pH of the solution to 7.95.

### Velocity Sedimentation Experiments on B28-N$^{\varepsilon}$-Myristoyl-Lys$^{B28}$Pro$^{B29}$

Velocity Sedimentation Experiments were performed on a Beckman XLI analytical ultracentrifuge. Samples and diluents of about 0.42 mL were loaded in 12 mm column cells with sapphire windows. The same volume of sample and diluent ensured that the meniscus on both the reference and sample side were matched. The samples were sedimented at a rotor speed of 50,000 rpm at 22°C. As the samples were being sedimented, a radial concentration gradient formed. The rate of the boundary movement was monitored by interference optics. Data sets of radial scans were collected at one minute intervals. The velocity data were analyzed by the whole boundary approach to generate a g(s*) versus s* profile, where s* is the sedimentation coefficient and g(s*) is a distribution function [see Stafford III, W. F., *Analytical Biochemistry*, *203,* 295-301 (1992)]. The g(s*) profile shows the hydrodynamic size distribution of different species present in the solution analyzed. For an ideal monodisperse system, the g(s*) profile is, to within a very good approximation, a Gaussian distribution. Thus, the diffusion coefficient of the macromolecule is related to the standard deviation of the g(s*) profile by the following equation:

$$D = (\sigma R_m \omega^2 t)^2 / 2t$$

Where D is the diffusion coefficient in $cm^2$/second;

$\sigma$ is the standard deviation (the peak width) of the g(s*) profile determined by fitting to a Gaussian distribution;

$R_m$ is the radius of the meniscus in cm;

$\omega$ is the velocity of rotation in radians per second; and

t is the time of the harmonic mean of all the scans used to analyze the data in seconds.

After the diffusion coefficient was calculated, the molecular weight of the species was calculated using the Svedberg equation shown below:

$$s/D = M(1 - \bar{v} \rho)/RT$$

where s is the sedimentation coefficient (peak position) in seconds;

D is the calculated diffusion coefficient in $cm^2$/second;

M is the molecular weight in grams/mole;

v is the partial specific volume of the monoacylated monomeric human insulin analog or monoacylated human insulin analog in mL/gram ( = 0.73 mL/g for the present analysis);

$\rho$ is the density of the solvent in grams/mL (density = 1 gram/mL for this analysis;

R is the gas constant ($8.31 \times 10^7$ erg/mole/degree K); and

T is the absolute temperature in Kelvins.

Figure 1 is a graphical representation of the sedimentation coefficient distribution profiles for formulations of B28-N$^\varepsilon$-myristoyl-Lys$^{B28}$Pro$^{B29}$ containing 2 moles of $Zn^{2+}$ per 6 moles of acylated monomeric human insulin analog, glycerine and phenol on a Beckman XLI analytical ultracentrifuge. The formulations are buffered with dibasic sodium phosphate at pH 7.95 ($\Delta$); buffered with Tris at pH 7.96 ($\square$); buffered with dibasic sodium phosphate at pH 7.5 ($\lozenge$); and buffered with Tris at pH 7.52 (no marker). The graph demonstrates the effect pH has on the association properties of acylated human insulin analogs. At a pH of about 7.5 the sedimentation velocity profiles indicate heterogeneous formulations. In direct contrast, at a pH of between about 7.9 and about 8.0, the sedimentation velocity profiles indicate a monodisperse hexameric association of the monoacylated monomeric human insulin analog. The graph also demonstrates the formation of a formulation containing an acylated monomeric human insulin analog in a monodisperse hexameric association state in two different buffer systems. The molecular weights calculated from the sedimentation velocity profiles for the monodisperse hexameric association formulations are presented in Table 2 and are indicative of a monodisperse hexamer association. The molecular weight for B28-N$^\varepsilon$-myristoyl-Lys$^{B28}$Pro$^{B29}$ human insulin analog is 6,018 grams/mole, and the theoretical molecular weight of a hexamer of B28-N$^\varepsilon$-myristoyl-Lys$^{B28}$Pro$^{B29}$ human insulin analog is 36,108 grams/mole. The calculated molecular weights presented in Table 2 are consistent with a hexameric association.

Table 2.

| Data obtained from sedimentation velocity experiments for monodisperse hexameric association formulations of B28-N$^\varepsilon$-myristoyl- Lys$^{B28}$Pro$^{B29}$. | | | | | |
|---|---|---|---|---|---|
| Buffer system | pH | s* peak (seconds) | Rotor Speed | Temperature (degrees Kelvin) | Calculated MW of association |
| 7 mM phosphate (Example 4) | 7.95 | $3.26 \times 10^{-13}$ | 50,000 rpm | 295 | 34,300 g/mole |
| 10 mM Tris (Example 2) | 7.96 | $3.52 \times 10^{-13}$ | 50,000 rpm | 295 | 35,000 g/mole |

The data collected from the sedimentation velocity experiments described above was also analyzed using the software DCDT Plus Version 1.05 available from Alliance Protein Laboratories (Camarillo, CA). Although the values can be calculated by hand as described above, the algorithm in the software allows values to be obtained more efficiently. The algorithm calculates a sedimentation coefficient distribution function which is fitted to yield concentration, sedimentation coefficient and diffusion coefficient or molecular mass. It has been shown in the literature that this method of analysis typically underestimates the diffusion coefficient and thus overestimates the molecular mass by a few percent. Values were calculated for B28-N$^\varepsilon$-myristoyl-Lys$^{B28}$Pro$^{B29}$ human insulin analog using the DCDT Plus Version 1.05 software and compared to those obtained by the method described above. At a pH of 7.5, the g(s*) distribution function clearly indicated heterogeneous aggregation phenomenon with large sedimentation coefficients. On the other hand, at a pH of 8.0, a model with sedimentation coefficient of 3.3 Svedberg, diffusion coefficient of 8.8 Fick or Molecular weight of 34,400 grams/mole was obtained and found to describe over 96% of the data. The molecular mass was calculated using the same solvent density (1.0 g/mL) and a solute partial specific volumes (0.73 g/mL). The results are consistent with those values calculated by hand using the above formula. Thus, the DCDT Plus Version 1.05 software was used to analyze the data on the other example solutions.

The standard procedure used to determine the state of association of the acylated monomeric insulin analogs or acylated insulin analogs in each of the experiments is described by the following steps. First, the sedimentation distribution function was calculated using the data from the sedimentation velocity experiments as described above. Next, the sedimentation distribution function was fitted to a single Gaussian function. If the single Gaussian function fit the data well as determined by inspection of the residual plots, then the association state was determined. If the fit was good, and the calculated molecular weight was approximately 32,000 grams/mole to 40,000 grams/mole, then this showed that hexamer was present. In order for the data to fit well to a single Gaussian function, the sample must be substantially homogeneous. As discussed above, a monodisperse hexameric association will exhibit a single well-defined peak in it sedimentation coefficient distribution profile which indicates that at least about 84% of the acylated monomeric insulin analog or the acylated insulin analog is present in a monodisperse hexameric association state.

If the fit obtained using the single Gaussian function is not good as judged by the residual plots, then the data was fit using two Gaussian functions which assumes that there are at least two different species present in the solution. The relative contribution from the different species is given by the ratio of the concentration and is calculated by the respective areas under the curves. If the data is not fit well using the two Gaussian functions, it was determined that the level of heterogeneity in the system is such that there is no dominant species in the solution. If the single Gaussian function fits the data, but the distribution is very broad, then this is another indication of heterogeneity in the system. The area under the curve is then compared to a hypothetical Guassian that represents a hexamer population. The ratio of the two provides an indication of the percentage of hexamer that may be present.

### Static Light Scattering Experiments

The association states of B28-N$^\varepsilon$-myristoyl-Lys$^{B28}$Pro$^{B29}$ was analyzed using static light scattering experiments to confirm hexamer formation. A formulation of B28-N$^\varepsilon$-myristoyl-Lys$^{B28}$Pro$^{B29}$ was prepared at a concentration of 3.6 mg/mL by reconstituting lyophilized vials prepared as described above with a diluent solution comprising 7 mM dibasic sodium phosphate, 16 mg/mL glycerin, and 5 mg/mL of phenol and then adjusting the pH to the desired level for the experiment by addition of hydrochloric acid or sodium hydroxide. Finally, the prepared solution was filtered through a 0.22 micron Millex-GV low protein binding filter to produce the sample for the experiment.

Static light scattering measurements were performed using a Brookhaven Instruments 9000AT autocorrelator and goniometer. All measurements were made with a 1 mm pinhole at 6 scattering angles and 4 replicates using a Lexel model 3500 argon ion laser at 488 nm. During the experiment, the temperature was maintained at 25°C using a Haake F3 temperature bath, and the scattering signal was calibrated at 90° using 0.2 micron filtered toluene with a known value of Rayleigh ratio ($R_\theta$). Weight-average molecular weights were then calculated using standard equations known to those skilled in the art [see Cantor, C. R. and Schimmel, P. R., BIOPHYSICAL CHEMISTRY, W. H. Freeman and Company, New York, New York, 838-843 (1980)]. In the analysis, a value of 0.183 mL/g, a number known to those skilled in the art, was used to reflect the change in refractive index per change in concentration of protein. Additionally, a value of 1.333 was used for the refractive index of water. The weight-average molecular weight ($M_w$) was calculated at various pH levels and is presented in Table 3. The weight-average molecular weights obtained from these studies at pH 8.0 and 8.3 are consistent with those expected for formation of hexameric associations.

Table 3.

| Molecular weights calculated from static light-scattering experiments for solutions of B28-N$^\varepsilon$-myristoyl-Lys$^{B28}$Pro$^{B29}$ at various pH levels. | | |
|---|---|---|
| pH | $M_w$ (g/mole) | Standard Deviation (g/mole) |
| 7.5 | 126,800 | 3,200 |

Table 3. (continued)

| Molecular weights calculated from static light-scattering experiments for solutions of B28-$N^\epsilon$-myristoyl-Lys$^{B28}$Pro$^{B29}$ at various pH levels. | | |
|---|---|---|
| pH | $M_w$ (g/mole) | Standard Deviation (g/mole) |
| 7.8 | 60,700 | 3,500 |
| 8.0 | 40,400 | 700 |
| 8.3 | 40,400 | 700 |
| 8.5 | 49,100 | 1,400 |

### EXAMPLE 5

**Preparation of B29-$N^\epsilon$-Octanoyl-Asp$^{B28}$ Human Insulin Analog Formulation at a pH of 7.5 and 8.0 with Phosphate Buffer**

To a first vial containing 80 mL of sterile water was added 3.2 mg synthetic glycerine, 1.13 g of liquefied phenol, and 365 mg of dibasic sodium phosphate. After dissolution, sterile water was added to produce a final weight of 100 grams for the mixture. The pH of the solution was measured and found to be 8.0.

To a second vial containing 4 mg of B29-$N^\epsilon$-octanoyl-Asp$^{B28}$ human insulin analog was added 0.5 mL of 0.1 N hydrochloric acid. After the protein had dissolved, 17.3 µL of I mg/mL ZnO stock solution was added. Finally, 0.5 mL of the solution from the first vial was added and the pH was adjusted to a final value of 7.5 using 1 N sodium hydroxide.

To a third vial was added 0.5 mL of the solution in the second vial adjusted to a pH of 7.5. The pH was then adjusted to a final value of 8.0 by adding 1 N sodium hydroxide.

A sample of about 0.45 mL of the solution at a pH of 7.5 in the second vial was added to a centrifuge cell and run at 60,000 rpm and 22°C on the Beckman XLI analytical ultracentrifuge. The experiment was repeated using about 0.45 mL of the solution at a pH of 8.0 in the third vial. The data was analyzed by DCDT Plus Version 1.05 as described above. A single Gaussian distribution did not fit the data well indicating that the system was not composed entirely of a single homogeneous population species. Thus, the fitting results using the single Gaussian distribution suggests some degree of heterogeneity in the system. The data was fit using two Gaussian distribution functions. For the solution at pH 7.5, a model with sedimentation coefficients of 3.0 Svedberg and a molecular weight of 27,700 g/mole was found to adequately describe over 92% of the data. The low molecular weight calculated indicates that hexamer is not a dominant species in the solution at a pH of 7.5. For the solution at pH 8.0, a model with sedimentation coefficient of 3.0 Svedberg and a molecular weight of 33,200 g/mole was found to adequately describe 85% of the data indicating that hexamer is the dominant species in solution. The remaining 15 % corresponds to species smaller than a hexamer. Thus, by shifting the pH from 7.5 to 8.0, the solution state can be converted to a substantially hexameric association state, *i.e.,* one that is at least 85% hexamer.

### EXAMPLE 6

**Preparation of B28-$N^\epsilon$-Octanoyl-Lys$^{B28}$Pro$^{B29}$ Human Insulin Analog Formulation at a pH of 7.4 and 8.0 with Phosphate Buffer**

To a first vial containing 80 mL of sterile water was added 3.2 mg synthetic glycerine, 1.13 g of liquefied phenol, and 365 mg of dibasic sodium phosphate. After dissolution, sterile water was added to produce a final weight of 100 grams for the mixture. The pH of the solution was measured and found to be 8.0.

To a second vial was added 8.3 mg of B28-$N^\epsilon$-octanoyl-Lys$^{B28}$Pro$^{B29}$ human insulin analog and 1 mL of 0.01 N hydrochloric acid. 35 µL of 1 mg/mL zinc oxide stock solution was then added to the second vial. Next, 1 mL of the solution from the first vial was added to the second vial and the pH was measured to be around 4.4. The pH was then adjusted to a final value of 7.4 using 1 N sodium hydroxide.

1 mL of the solution at pH 7.4 was pipetted into a third vial and the pH was adjusted to a final pH of 8.0 using 1 N sodium hydroxide.

A sample of about 0.45 mL of the solution at a pH of 7.4 in the second vial was added to a centrifuge cell and run at 60,000 rpm and 22°C on the Beckman XLI analytical ultracentrifuge. The experiment was repeated using about 0.45 mL of the solution at a pH of 8.0 in the third vial. The data was analyzed by DCDT Plus Version 1.05 as described above. For the solution at pH 7.4, neither a single nor two species model fits the data well as judged by the residual plots and no reliable molecular weight measurements could be obtained indicating a significant amount of heterogeneity in the system.

On the other hand, attempts to fit the data from the solution at pH 8.0 gave fitting parameters of 2.9 Svedberg and a molecular weight of 35,300 g/mole. The fitting of the data, for the solution at pH 8.0 could be further refined to provide a model with a sedimentation coefficient of 3.0 Svedberg and a molecular weight of 40,800 g/mole. This was found to describe over 94% of the data indicating that the hexameric species was the dominant species in solution. Although there might be some hexamer present at a pH of 7.4, the studies indicate that this is not a dominant species at this pH. However, at a pH of 8.0, the solution state is composed of substantially the hexameric association state.

## EXAMPLE 7

**Preparation of B29-N$^\varepsilon$-Myristoyl-des(B30) Human Insulin Formulation at a pH of 7.5, 7.97, and 8.4 with Phosphate Buffer**

To a first vial containing 80 mL of sterile water was added 3.5 mg synthetic glycerine, 1.13 g of liquefied phenol, and 377 mg of dibasic sodium phosphate. After dissolution, sterile water was added to produce a final weight of 100 grams for the mixture. The pH of the solution was measured and found to be 8.0.

To a second vial containing 4.2 mg of B29-N$^\varepsilon$-myristoyl-des(B30) human insulin was added 0.5 mL of 0.1 N hydrochloric acid. The acylated protein content of the B29-N$^\varepsilon$-myristoyl-des(B30) human insulin was about 80% (w/w) and 66% (w/w) of the protein was acylated at the correct site. After the protein had dissolved, 17.3 µL of 1 mg/mL ZnO stock solution was added. Finally, 0.5 mL of the solution from the first vial was added and the pH was adjusted to a final value of 7.5 using 1 N sodium hydroxide.

To a third vial was added 0.5 mL of the solution from the second vial adjusted to a pH of 7.5. The pH was then adjusted to a final value of 8.4 by adding I N sodium hydroxide.

1.8 mg of B29-N$^\varepsilon$-myristoyl-des(B30) human insulin was dissolved in 0.25 mL 0.1 N hydrochloric acid. 9 µL of a 1 mg/mL zinc oxide stock solution was added to the insulin solution and then a 0.25 mL of a diluent was added that contained 14 mM dibasic sodium phosphate; 32 mg/mL glycerin and 10mg/mL phenol. The pH was then adjusted to a value of 7.97 using 2 N sodium hydroxide.

A sample of about 0.45 mL of the solution at a pH of 7.5 in the second vial was added to a centrifuge cell and run at 60,000 rpm and 22°C on the Beckman XLI analytical ultracentrifuge. The experiment was repeated using about 0.45 mL of the solution at a pH of 8.4 in the third vial. The experiment was also repeated using about 0.45 mL of the solution at a pH of 7.97. The data was analyzed by DCDT Plus Version 1.05 as described above. The g(s*) distribution functions are very different at the three different pHs. The peak positions are shifted to higher values at lower pH. Additionally, the distribution function is significantly broader at lower pH suggesting increased heterogeneity in the system. Fitting the data to a single Gaussian distribution function gave acceptable results so that the sedimentation profiles appear to be adequately described using the single Gaussian distribution. The fitted parameters are summarized in the following Table and shown in Figure 2 which illustrates that the peaks narrow and are shifted to lower values as the pH is increased from 7.5.

Table 4.

| Data obtained from sedimentation velocity experiments for solutions of B29-N$^\varepsilon$-myristoyl-des(B30) human insulin at various pHs. | | | |
|---|---|---|---|
| Solution pH | s (Svedberg) | Diffusion (Fick) | MW (g/mole) |
| pH 7.5 | 5.7 | 22.6 | 23,000 |
| pH 7.97 | 4.5 | 13.4 | 30,700 |
| pH 8.4 | 4.2 | 9.8 | 39,000 |

The data at pH 7.5 indicates the presence of heterogeneity or intermediates in the system. It is very unlikely for a molecule with a sedimentation coefficient of 5.7 Svedberg to have such mobility with a a diffusion coefficient of 22.6 Ficks. By shifting to higher pH, the heterogeneity in the system was significantly reduced as described above. By shifting the pH to 8.0, a majority, estimated at 92 percent or greater, of the acylated insulin analog was in the hexamer association state as determined by comparing the area of the observed sedimentation profile to a curve representing an ideal hypothetical hexamer population. By shifting the pH to 8.4, the fitting results indicated that even more of the acylated insulin is in the hexameric association state and heterogeneity was significantly reduced.

The present invention may be embodied in other specific forms without departing from its spirit or its central characteristics. The described embodiments are to be considered in all respects only as illustrative and not restrictive. The scope of the invention is, therefore, indicated by the appended claims, rather than by the foregoing description. All changes which come within the meaning and range of equivalency of the claims are to be embraced within their scope.

**Claims**

1. A pharmaceutical formulation suitable for administration to a patient, comprising: an aqueous solution with a pH of greater than about 7.9, the aqueous solution comprising:

   a) an isotonicity agent;
   b) a phenolic derivative;
   c) zinc ions; and
   d) a monoacylated human insulin analog or a pharmaceutically acceptable salt of the monoacylated human insulin analog, the monoacylated human insulin analog or the pharmaceutically acceptable salt of the monoacylated human insulin analog comprising the polypeptide of SEQ ID NO: 1 properly cross-linked to SEQ ID NO.2, wherein

   Xaa at position 21 of SEQ ID NO: 1 is selected from the group consisting of Asn, Asp, Gly, and Glx;
   Xaa at position of 3 of SEQ ID NO: 2 is selected from the group consisting of Asn, Asp, and Glx;
   Xaa at position 28 of SEQ ID NO:2 is selected from the group consisting of Asp, Leu, Val, Ala, and acylated Lys; and
   Xaa at position 29 of SEQ ID NO:2 is selected from the group consisting of Pro and acylated Lys; and
   further wherein position 28 or position 29 is acylated Lys, and
   if position 28 is acylated Lys, position 29 is not acylated Lys, and
   if position 29 is acylated Lys, position 28 is not acylated Lys.

2. The pharmaceutical formulation according to claim 1, wherein there are 2.5 to 3 moles of zinc ion present per 6 moles of monoacylated human insulin analog.

3. The pharmaceutical formulation according to Claim 1, wherein there are 2 to 3 moles of zinc ion present per 6 moles of monoacylated human insulin analog.

4. The pharmaceutical formulation according to claim 1, wherein Xaa at position 3 of SEQ ID NO:2 is Asn.

5. The pharmaceutical formulation according to claim 1, wherein Xaa at position 21 of SEQ ID NO:1 is Asn and wherein Xaa at position 3 of SEQ ID NO:2 is Asn.

6. The pharmaceutical formulation according to claim 1, wherein the monoacylated human insulin analog is an acylated $Lys^{B28}Pro^{B29}$ human insulin.

7. The pharmaceutical formulation according to claim 1, wherein the monoacylated human insulin analog comprises an acyl group having from 6 to 21 carbon atoms.

8. The pharmaceutical formulation according to claim 7, wherein the acyl group is selected from the group consisting of myristoyl and palmitoyl.

9. The pharmaceutical formulation according to claim 1, wherein the monoacylated human insulin analog is selected from the group consisting of B28-N$^\varepsilon$-palmitoyl-$Lys^{B28}Pro^{B29}$ insulin, and B28-N$^\varepsilon$-myristoyl-$Lys^{B28}Pro^{B29}$ insulin.

10. The pharmaceutical formulation according to claim 1, wherein the monoacylated human insulin analog is acylated $Asp^{B28}$ insulin.

11. The pharmaceutical formulation according to claim 10, wherein the monoacylated human insulin analog is selected from the group consisting of B29-N$^\varepsilon$-palmitoyl-$Asp^{B28}$ human insulin, and B29-N$^\varepsilon$-myristoyl-$Asp^{B28}$ human insulin.

12. The pharmaceutical formulation according to claim 1, wherein the pH of the formulation is selected from the group consisting of 7.95, 7.96, 8.0, and 8.3.

13. The pharmaceutical formulation according to claim 1, wherein the pH of the formulation is about 8.0.

14. The pharmaceutical formulation according to claim 1, wherein the formulation has a pH of greater than 7.9 and less than 8.3.

15. The pharmaceutical formulation according to claim 1, wherein the pH of the aqueous solution is greater than 7.9 and less than 8.0.

16. The pharmaceutical formulation according to claim 1, further comprising a buffer.

17. The pharmaceutical formulation according to claim 16, wherein the buffer is selected from the group consisting of tris(hydroxymethyl)aminomethane, sodium phosphate, glycylglycine, citrate, and combinations thereof.

18. The pharmaceutical formulation according to claim 1, wherein the phenolic derivative is selected from the group consisting of phenol, m-cresol, o-cresol, p-cresol, chloro-cresol, benzyl alcohol, phenylethanol, phenoxyethanol, methylparaben, and combinations thereof.

19. The pharmaceutical formulation according to claim 18, wherein the phenolic derivative is selected from the group consisting of phenol, m-cresol, and combinations thereof.

20. The pharmaceutical formulation according to claim 1, wherein the isotonicity agent is selected from the group consisting of glycerine, sodium chloride, a carbohydrate, and combinations thereof.

21. The pharmaceutical formulation according to Claim 20, wherein the carbohydrate is selected from the group consisting of sucrose, dextrose, mannitol, trehalose, and combinations thereof.

22. Use of the pharmaceutical formulation according to claim 1 for the manufacture of a medicament for the treatment of a patient suffering from hyperglycemia.

23. A method of preparing a pharmaceutical formulation suitable for administration to a patient, comprising:

(a) contacting zinc or cobalt in the +2 oxidation state with a monoacylated human insulin analog, or a pharmaceutically acceptable salt of the analog, wherein the analog comprises the polypeptide of SEQ ID NO: 1 properly cross-linked to SEQ ID NO: 2, and wherein:

Xaa at position 21 of SEQ ID NO:1 is selected from the group consisting of Asn, Asp, Gly, and Glx;
Xaa at position 3 of SEQ ID NO:2 is selected from the group consisting of Asn, Asp, and Glx;
Xaa at position 28 of SEQ ID NO:2 is selected from the group consisting of Asp, Leu, Val, Ala, and acylated Lys;
Xaa at position 29 of SEQ ID NO:2 is selected from the group consisting of Pro and acylated Lys, and further wherein position 28 or position 29 is acylated Lys, and
if position 28 is acylated Lys, position 29 is not acylated Lys, and
if position 29 is acylated Lys, position 28 is not acylated Lys;

(b) adjusting the pH of the pharmaceutical formulation to greater than about 7.9; and
(c) adding a phenolic derivative and an isotonicity agent.

24. The method according to claim 23, wherein Xaa at position 21 of SEQ ID NO: 1 is Asn.

25. The method according to claim 23, wherein Xaa at position 3 of SEQ ID NO:2 is Asn.

26. The method according to claim 23, wherein Xaa at position 21 of SEQ ID NO: is Asn and wherein Xaa at position 3 of SEQ ID NO:2 is Asn.

27. The method according to claim 23, wherein the metal is zinc.

28. The method according to claim 23, wherein the phenolic derivative is selected from the group consisting of phenol, m-cresol, o-cresol, p-cresol, chloro-cresol, benzyl alcohol, phenylethanol, methylparaben, resorcinol, phenoxyethanol, and combinations thereof.

29. The method according to claim 28, wherein the phenolic derivative is selected from the group consisting of phenol, m-cresol, and combinations thereof.

30. The method according to claim 23, further comprising adding a buffer to the formulation.

31. The method according to claim 30, wherein the buffer is selected from the group consisting of tris (hydroxymethyl) aminomethane, sodium phosphate, glycylglycine, citrate, and combinations thereof.

32. The method according to claim 23, wherein the isotonicity agent is selected from the group consisting of glycerine, sodium chloride, a carbohydrate, and combinations thereof.

33. The method according to claim 32, wherein the carbohydrate is selected from the group consisting of sucrose, dextrose, mannitol, trehalose, and combinations thereof.

34. The method according to claim 23, further comprising lyophilizing the metal/analog mixture of step(a).

35. The method according to claim 23, wherein the pH is adjusted to a value of greater than 7.9 and less than 8.3.

36. The method according to claim 23, wherein the pH is adjusted to a value of greater than 7.9 and less than 8.0.

37. A pharmaceutical formulation suitable for administration to a patient comprising: an aqueous solution with a pH of greater than about 7.9, the aqueous solution comprising:

   (a) an isonicity agent;
   (b) a phenolic derivative;
   (c) zinc ions; and
   (d) a monoacylated des (B30) human insulin analog or pharmaceutically acceptable salt of the monoacylated des (B30) human insulin analog.

38. The pharmaceutical formulation according to claim 37, wherein there are 2.5 to 3 moles of zinc ion present per 6 moles of monoacylated human insulin analog.

39. The pharmaceutical formulation according to claim 37, wherein there are 2 to 3 moles of zinc ion present per 6 moles of monoacylated human insulin analog.

40. The pharmaceutical formulation according to claim 37, wherein the pH of the aqueous solution is between 7.9 and 8.4.

41. The pharmaceutical formulation according to claim 37, further comprising a buffer.

42. The pharmaceutical formulation according to claim 41, wherein the buffer is selected from the group consisting of tris(hydroxymethyl)aminomethane, sodium phosphate, glycylglycine, citrate, and combinations thereof.

43. The pharmaceutical formulation according to claim 37, wherein the phenolic derivative is selected from the group consisting of phenol, m-cresol, o-cresol, p-cresol, chloro-cresol, benzyl alcohol, phenylethanol, phenoxyethanol, methylparaben, and combinations thereof.

44. The pharmaceutical formulation according to claim 43, wherein the phenolic derivative is selected from the group consisting of phenol, m-cresol, and combinations thereof.

45. The pharmaceutical formulation according to claim 37, wherein the isotonicity agent is selected from the group consisting of glycerine, sodium chloride, a carbohydrate, and combinations thereof.

46. The pharmaceutical formulation according to claim 45, wherein the carbohydrate is selected from the group consisting of sucrose, dextrose, mannitol, trehalose, and combinations thereof.

47. The pharmaceutical formulation according to claim 37, wherein the monoacylated des (B30) human insulin analog comprises an acyl group having from 6 to 21 carbon atoms.

48. The pharmaceutical formulation according to claim 37, wherein the monoacylated des(B30) human insulin analog is selected from the group consisting of B29-$N^\varepsilon$-palmitoyl-des(B30) human insulin and B29-$N^\varepsilon$-myristoyl-des(B30) human insulin.

49. The pharmaceutical formulation according to claim 37, wherein the pH of the formulation is selected from the group consisting of 7.97 and 8.4.

50. Use of the pharmaceutical formulation according to claim 37 for the manufacture of a medicament for the treatment a patient suffering from hyperglycemia.

51. A method of preparing a pharmaceutical formulation suitable for administration to a patient, comprising:

(a) contacting zinc or cobalt in the +2 oxidation state with a monoacylated des(B30) human insulin analog or a pharmaceutically acceptable salt of the monoacylated des(B30) human insulin analog;
(b) adjusting the pH of the pharmaceutical formulation to greater than about 7.9; and
(c) adding a phenolic derivative and an isotonicity agent.

52. The method according to claim 51, wherein the metal is zinc.

53. The method according to claim 51, wherein the phenolic derivative is selected from the group consisting of phenol, m-cresol, o-cresol, p-cresol, chloro-cresol, benzyl alcohol, phenylethanol, methylparaben, resorcinol, phenoxyethanol, and combinations thereof.

54. The method according to claim 53, wherein the phenolic derivative is selected from the group consisting of phenol, m-cresol, and combinations thereof.

55. The method according to claim 51, further comprising adding a buffer to the formulation.

56. The method according to claim 55, wherein the buffer is selected from the group consisting of tris(hydroxymethyl) aminomethane, sodium phosphate, glycylglycine, citrate, and combinations thereof.

57. The method according to claim 51, wherein the isotonicity agent is selected from the group consisting of glycerine, sodium chloride, a carbohydrate, and combinations thereof.

58. The method according to claim 57, wherein the carbohydrate is selected from the group consisting of sucrose, dextrose, mannitol, trehalose, and combinations thereof.

59. The method according to claim 51, wherein the pH is adjusted to a value of between 7.9 and 8.4.

**Patentansprüche**

1. Pharmazeutische Formulierung, die zur Verabreichung an einen Patienten geeignet ist, welche eine wäßrige Lösung mit einem pH von mehr als etwa 7.9 umfaßt, wobei die wäßrige Lösung enthält:

a) ein Isotonizitätsmittel,
b) ein Phenolderivat
c) Zinkionen und
d) ein monoacyliertes Humaninsulinanalogon oder ein pharmazeutisch annehmbares Salz des monoacylierten Humaninsulinanalogons, wobei das monoacylierte Humaninsulinanalogon oder das pharmazeutisch annehmbare Salz des monoacylierten Humaninsulinanalogons das Polypeptid der SEQ ID Nr. 1 geeignet vernetzt mit SEQ ID Nr. 2 umfaßt, worin

Xaa an der Position 21 der SEQ ID Nr. 1 aus der Gruppe ausgewählt ist, die besteht aus Asn, Asp, Gly und Glx,
Xaa an der Position 3 der SEQ ID Nr. 2 aus der Gruppe ausgewählt ist, die besteht aus Asn, Asp und Glx,
Xaa an der Position 28 der SEQ ID Nr. 2 aus der Gruppe ausgewählt ist, die besteht aus Asp, Leu, Val, Ala und acyliertem Lys, und
Xaa an der Position 29 der SEQ ID Nr. 2 aus der Gruppe ausgewählt ist, die besteht aus Pro und acyliertem Lys, und
worin ferner die Position 28 oder Position 29 acyliertes Lys ist, und
falls Position 28 acyliertes Lys ist, die Position 29 kein acyliertes Lys ist, und
falls Position 29 acyliertes Lys ist, die Position 28 kein acyliertes Lys ist.

2. Pharmazeutische Formulierung nach Anspruch 1, worin 2,5 bis 3 Mol Zinkionen pro 6 Mol monoacyliertem Humaninsulinanalogon vorhanden sind.

3. Pharmazeutische Formulierung nach Anspruch 1, worin 2 bis 3 Mol Zinkionen pro 6 Mol monoacyliertem Humaninsulinanalogon vorhanden sind.

4. Pharmazeutische Formulierung nach Anspruch 1, worin Xaa an der Position 3 von SEQ ID Nr. 2 für Asn steht.

5. Pharmazeutische Formulierung nach Anspruch 1, worin Xaa an der Position 21 der SEQ ID Nr. 1 für Asn steht und worin Xaa an der Position 3 der SEQ ID Nr. 2 für Asn steht.

6. Pharmazeutische Formulierung nach Anspruch 1. worin das monoacylierte Humaninsulinanalogon ein acyliertes $Lys^{B28}Pro^{B29}$ Humaninsulin ist.

7. Pharmazeutische Formulierung nach Anspruch 1, worin das monoacylierte Humaninsulinanalogon eine Acylgruppe mit 6 bis 21 Kohlenstoffatomen umfaßt.

8. Pharmazeutische Formulierung nach Anspruch 7, worin die Acylgruppe aus der Gruppe ausgewählt ist, die aus Myristoyl und Palmitoyl besteht.

9. Pharmazeutische Formulierung nach Anspruch 1, worin das monoacylierte Humaninsulinanalogon aus der Gruppe ausgewählt ist, die besteht aus B28-$N^{\varepsilon}$-Palmitoyl-$Lys^{B28}Pro^{B29}$ Insulin und B28-$N^{\varepsilon}$-Myristoyl-$Lys^{B28}Pro^{B29}$ Insulin.

10. Pharmazeutische Formulierung nach Anspruch 1, worin das monoacylierte Humaninsulinanalogon acyliertes $Asp^{B28}$ Insulin ist.

11. Pharmazeutische Formulierung nach Anspruch 10, worin das monoacylierte Humaninsulinanalogon aus der Gruppe ausgewählt ist, die besteht aus B29-$N^{\varepsilon}$-Palmitoyl-$Asp^{B28}$ Humaninsulin und B29-$N^{\varepsilon}$-Myristoyl-$Asp^{B28}$ Humaninsulin.

12. Pharmazeutische Formulierung nach Anspruch 1, worin der pH der Formulierung aus der Gruppe ausgewählt ist, die besteht aus 7,95, 7.6. 8.0 und 8.3.

13. Pharmazeutische Formulierung nach Anspruch 1, worin der pH der Formulierung etwa 8,0 beträgt.

14. Pharmazeutische Formulierung nach Anspruch 1, worin die Formulierung einen pH von mehr als 7,9 und weniger als 8,3 aufweist.

15. Pharmazeutische Formulierung nach Anspruch 1, worin der pH der wäßrigen Lösung mehr als 7.9 und weniger als 8,0 beträgt.

16. Pharmazeutische Formulierung nach Anspruch 1, die ferner einen Puffer enthält.

17. Pharmazeutische Formulierung nach Anspruch 16, worin der Puffer aus der Gruppe ausgewählt ist, die besteht aus Trishydroxymethylaminomethan, Natriumphosphat, Glycylglycin, Citrat und Kombinationen hiervon.

18. Pharmazeutische Formulierung nach Anspruch 1, worin das Phenolderivat aus der Gruppe ausgewählt ist, die besteht aus Phenol, m-Cresol, o-Cresol, p-Cresol, Chlorcresol, Benzylalkohol, Phenylethanol, Phenoxyethanol, Methylparaben und Kombinationen hiervon.

19. Pharmazeutische Formulierung nach Anspruch 18, worin das Phenolderivat aus der Gruppe ausgewählt ist, die besteht aus Phenol, m-Cresol und Kombinationen hiervon.

20. Pharmazeutische Formulierung nach Anspruch 1, worin das Isotonizitätsmittel aus der Gruppe ausgewählt wird, die besteht aus Glycerin, Natriumchlorid, einem Kohlenhydrat und Kombinationen hiervon.

21. Pharmazeutische Formulierung nach Anspruch 20, worin das Kohlenhydrat aus der Gruppe ausgewählt ist, die besteht aus Saccharose, Dextrose, Mannit, Trehalose und Kombinationen hiervon.

22. Venvendung der pharmazeutischen Formulierung nach Anspruch 1 zur Herstellung eines Arzneimittels zur Behandlung eines Patienten, der an Hyperglycämie leidet.

23. Verfahren zur Herstellung einer pharmazeutischen Formulierung, die zur Verabreichung an einen Patienten geeignet ist, **gekennzeichnet durch**

(a) Zusammenbringen von Zink oder Cobalt in der Oxidationstufe +2 mit einem monoacylierten Humaninsulinanalogon oder einem pharmazeutisch annehmbaren Salz des Analogons, worin das Analogon das Polypeptid der SEQ ID Nr. 1 geeignet vernetzt mit SEQ ID Nr. 2 umfaßt, worin Xaa an der Position 21 der SEQ ID Nr. 1 aus der Gruppe ausgewählt ist, die besteht aus Asn, Asp, Gly und Glx,
Xaa an der Position 3 der SEQ ID Nr. 2 aus der Gruppe ausgewählt ist, die besteht aus Asn, Asp und Glx,
Xaa an der Position 28 der SEQ ID Nr. 2 aus der Gruppe ausgewählt ist, die besteht aus Asp, Leu, Val, Ala und acyliertem Lys,
Xaa an der Position 29 der SEQ ID Nr. 2 aus der Gruppe ausgewählt ist, die besteht aus Pro und acyliertem Lys, und
worin ferner die Position 28 oder Position 29 acyliertes Lys ist, und
falls Position 28 acyliertes Lys ist, die Position 29 kein acyliertes Lys ist, und
falls Position 29 acyliertes Lys ist, die Position 28 kein acyliertes Lys ist.

(b) Einstellen des pH der pharmazeutischen Formulierung auf mehr als etwa 7,9, und

(c) Zugabe eines Phenolderivats und eines Isotonizitätsmittels.

24. Verfahren nach Anspruch 23, worin Xaa an Position 21 der SEQ ID Nr. 1 für Asn steht.

25. Verfahren nach Anspruch 23, worin Xaa an Position 3 der SEQ ID Nr. 2 für Asn steht.

26. Verfahren nach Anspruch 23, worin Xaa an Position 21 der SEQ ID Nr. 1 für Asn steht und worin Xaa an Position 3 der SEQ ID Nr. 2 für Asn steht.

27. Verfahren nach Anspruch 23, worin das Metall Zink ist.

28. Verfahren nach Anspruch 23, worin das Phenolderivat aus der Gruppe ausgewählt ist, die besteht aus Phenol, m-Cresol, o-Cresol, p-Cresol, Chlorcresol, Benzylalkohol, Phenylethanol, Methylparaben, Resorcin, Phenoxyethanol und Kombinationen hiervon.

29. Verfahren nach Anspruch 28, worin das Phenolderivat aus der Gruppe ausgewählt ist, die besteht aus Phenol, m-Cresol und Kombinationen hiervon.

30. Verfahren nach Anspruch 23, das ferner die Zugabe eines Puffers zur Formulierung umfaßt.

31. Verfahren nach Anspruch 30, worin der Puffer aus der Gruppe ausgewählt ist, die besteht aus Trishydroxymethylaminomethan, Natriumphosphat, Glycylglycin, Citrat und Kombinationen hiervon.

32. Verfahren nach Anspruch 23, worin das Isotonizitätsmittel aus der Gruppe ausgewählt ist, die besteht aus Glycerin, Natriumchlorid, einem Kohlenhydrat und Kombinationen hiervon.

33. Verfahren nach Anspruch 32, worin das Kohlenhydrat aus der Gruppe ausgewählt ist, die besteht aus Saccharose, Dextrose, Mannit, Trehalose und Kombinationen hiervon.

**34.** Verfahren nach Anspruch 23, das ferner die Lyophilisierung des Metall/Analogongemisches von Schritt (a) umfaßt.

**35.** Verfahren nach Anspruch 23, worin der pH auf einen Wert von mehr als 7.9 und weniger als 8,3 eingestellt wird.

**36.** Verfahren nach Anspruch 23, worin der pH auf einen Wert von mehr als 7.9 und weniger als 8.0 eingestellt wird.

**37.** Pharmazeutische Formulierung, die zur Verabreichung an einen Patienten geeignet ist, welche eine wäßrige Lösung mit einem pH von mehr als etwa 7.9 umfaßt, wobei die wäßrige Lösung enthält:

      (a) ein Isotonizitätsmittel,
      (b) ein Phenolderivat,
      (c) Zinkionen, und
      (d) ein monoacyliertes Des(B30)-Humaninsulinanalogon oder ein pharmazeutisch annehmbares Salz des monoacylierten Des(B30)-Humaninsulinanalogons.

**38.** Pharmazeutische Formulierung nach Anspruch 37, worin 2.5 bis 3 Mol Zinkionen pro 6 Mol monoacyliertem Insulinanalogon vorhanden sind.

**39.** Pharmazeutische Formulierung nach Anspruch 37, worin 2 bis 3 Mol Zinkionen pro 6 Mol monoacyliertem Insulinanalogon vorhanden sind.

**40.** Pharmazeutische Formulierung nach Anspruch 37, worin der pH der wäßrigen Lösung zwischen 7.9 und 8.4 liegt.

**41.** Pharmazeutische Formulierung nach Anspruch 37, die ferner einen Puffer enthält.

**42.** Pharmazeutische Formulierung nach Anspruch 41, worin der Puffer aus der Gruppe ausgewählt ist, die besteht aus Trishydroxymethylaminomethan, Natriumphosphat, Glycylglycin, Citrat und Kombinationen hiervon.

**43.** Pharmazeutische Formulierung nach Anspruch 37, worin das Phenolderivat aus der Gruppe ausgewählt ist, die besteht aus Phenol, m-Cresol, o-Cresol, p-Cresol, Chlorcresol, Benzylalkohol, Phenylethanol, Phenoxyethanol, Methylparaben und Kombinationen hiervon.

**44.** Pharmazeutische Formulierung nach Anspruch 43, worin das Phenolderivat aus der Gruppe ausgewählt ist, die besteht aus Phenol, m-Cresol und Kombinationen hiervon.

**45.** Pharmazeutische Formulierung nach Anspruch 37, worin das Isotonizitätsmittel aus der Gruppe ausgewählt ist, die besteht aus Glycerin, Natriumchlorid, einem Kohlenhydrat und Kombinationen hiervon.

**46.** Pharmazeutische Formulierung nach Anspruch 45, worin das Kohlenhydrat aus der Gruppe ausgewählt ist, die besteht aus Saccharose, Dextrose, Mannit, Trehalose und Kombinationen hiervon.

**47.** Pharmazeutische Formulierung nach Anspruch 37, worin das monoacylierte Des(B30)-Humaninsulinanalogon eine Acylgruppe mit 6 bis 21 Kohlenstoffatomen umfaßt.

**48.** Pharmazeutische Formulierung nach Anspruch 37, worin das monoacylierte Des(B30)-Humaninsulinanalogon aus der Gruppe ausgewählt ist, die besteht aus B29-N$^{\varepsilon}$-Palmitoyl—des(B30)-Humaninsulin, und B29-N$^{\varepsilon}$-Myristoyldes(B30)-Humaninsulin.

**49.** Pharmazeutische Formulierung nach Anspruch 37, worin der pH der Formulierung aus der Gruppe ausgewählt ist. die besteht aus 7,97 und 8,4.

**50.** Venvendung der pharmazeutischen Formulierung nach Anspruch 37 zur Herstellung eines Arzneimittels zur Behandlung eines Patienten, der an Hyperglycämie leidet.

**51.** Verfahren zur Herstellung einer pharmazeutischen Formulierung, die zur Verabreichung an einen Patienten geeignet ist, **gekennzeichnet durch**

      (a) Zusammenbringen von Zink oder Cobalt in der Oxidationstufe +2 mit einem monoacylierten Des(B30)-Humaninsulinanalogon oder einem pharmazeutisch annehmbaren Salz des monoacylierten Des(B30)-Humaninsulinanalogons,

(b) Einstellen des pH der pharmazeutischen Formulierung auf mehr als etwa 7,9, und

(c) Zugabe eines Phenolderivats und eines Isotonizitätsmittels.

52. Verfahren nach Anspruch 51, worin das Metall Zink ist.

53. Verfahren nach Anspruch 51, worin das Phenolderivat aus der Gruppe ausgewählt ist, die besteht aus Phenol, m-Cresol, o-Cresol, p-Cresol, Chlorcresol, Benzylalkohol, Phenylethanol, Methylparaben, Resorcin, Phenoxyethanol und Kombinationen hiervon.

54. Verfahren nach Anspruch 53, worin das Phenolderivat aus der Gruppe ausgewählt ist die besteht aus Phenol, m-Cresol und Kombinationen hiervon.

55. Verfahren nach Anspruch 51, das ferner die Zugabe eines Puffers zur Formulierung umfaßt.

56. Verfahren nach Anspruch 55, worin der Puffer aus der Gruppe ausgewählt ist, die besteht aus Trishydroxymethylaminomethan, Natriumphosphat, Glycylglycin, Citrat und Kombinationen hiervon.

57. Verfahren nach Anspruch 51, worin das Isotonizitätsmittel aus der Gruppe ausgewählt ist, die besteht aus Glycerin, Natriumchlorid, einem Kohlenhydrat und Kombinationen hiervon.

58. Verfahren nach Anspruch 57, worin das Kohlenhydrat aus der Gruppe ausgewählt ist, die besteht aus Saccharose, Dextrose, Mannit, Trehalose und Kombinationen hiervon.

59. Verfahren nach Anspruch 51, worin der pH auf einen Wen zwischen 7,9 und 8,4 eingestellt wird.

**Revendications**

1. Formulation pharmaceutique appropriée pour une administration à un patient, comprenant une solution aqueuse dont le pH est supérieur à environ 7,9, la solution aqueuse comprenant :

   a) un agent d'isotonicité ;
   b) un dérivé phénolique ;
   c) des ions de zinc ; et
   d) un analogue monoacylé de l'insuline humaine ou un sel pharmaceutiquement acceptable de l'analogue monoacylé de l'insuline humaine, l'analogue monoacylé de l'insuline humaine ou le sel pharmaceutiquement acceptable de l'analogue monoacylé de l'insuline humaine comprenant le polypeptide de SEQ ID NO: 1 réticulé de manière appropriée à SEQ ID NO: 2, dans laquelle

   Xaa à la position 21 de SEQ ID NO: 1 est choisi parmi le groupe constitué par Asn, Asp, Gly et Glx ;
   Xaa à la position 3 de SEQ ID NO: 2 est choisi parmi le groupe constitué par Asn, Asp et Glx ;
   Xaa à la position 28 de SEQ ID NO: 2 est choisi parmi le groupe constitué par Asp, Leu, Val, Ala, et Lys acylé;
   Xaa à la position 29 de SEQ ID NO: 2 est choisi parmi le groupe constitué par Pro et Lys acylé ; et
   en outre, dans laquelle, la position 28 ou la position 29 représente Lys acylé ; et
   si la position 28 représente Lys acylé, la position 29 ne représente pas Lys acylé, et
   si la position 29 représente Lys acylé, la position 28 ne représente pas Lys acylé.

2. Formulation pharmaceutique selon la revendication 1, dans laquelle de 2,5 à 3 moles d'ions de zinc sont présents par 6 moles de l'analogue monoacylé de l'insuline humaine.

3. Formulation pharmaceutique selon la revendication 1, dans laquelle de 2 à 3 moles d'ions de zinc sont présents par 6 moles de l'analogue monoacylé de l'insuline humaine.

4. Formulation pharmaceutique selon la revendication 1, dans laquelle Xaa à la position 3 de SEQ ID NO: 2 représente Asn.

5. Formulation pharmaceutique selon la revendication 1, dans laquelle Xaa à la position 21 de SEQ ID NO: 1 représente Asn et dans laquelle Xaa à la position 3 de SEQ ID NO: 2 représente Asn.

6. Formulation pharmaceutique selon la revendication 1, dans laquelle l'analogue monoacylé de l'insuline humaine est une insuline humaine $Lys^{B28}Pro^{B29}$ acylée.

7. Formulation pharmaceutique selon la revendication 1, dans laquelle l'analogue monoacylé de l'insuline humaine comprend un groupe acyle contenant de 6 à 21 atomes de carbone.

8. Formulation pharmaceutique selon la revendication 7, dans laquelle le groupe acyle est choisi parmi le groupe constitué par un groupe myristoyle et par un groupe palmitoyle.

9. Formulation pharmaceutique selon la revendication 1, dans laquelle l'analogue monoacylé de l'insuline humaine est choisi parmi le groupe constitué par l'insuline $B28$-$N^\epsilon$-palmitoyl-$Lys^{B28}Pro^{B29}$ et par l'insuline $B28$-$N^\epsilon$-myristoyl-$Lys^{B28}Pro^{B29}$.

10. Formulation pharmaceutique selon la revendication 1, dans laquelle l'analogue monoacylé de l'insuline humaine est l'insuline $Asp^{B28}$ acylée.

11. Formulation pharmaceutique selon la revendication 10, dans laquelle l'analogue monoacylé de l'insuline humaine est choisi parmi le groupe constitué par l'insuline humaine $B29$-$N^\epsilon$-palmitoyl-$Asp^{B28}$ et par l'insuline humaine $B29$-$N^\epsilon$-myristoyl-$Asp^{B28}$.

12. Formulation pharmaceutique selon la revendication 1, dans laquelle le pH de la formulation est choisi parmi le groupe constitué par 7,95, 7,96, 8,0 et 8,3.

13. Formulation pharmaceutique selon la revendication 1, dans laquelle le pH de la formulation s'élève à environ 8,0.

14. Formulation pharmaceutique selon la revendication 1, dans laquelle la formulation possède un pH supérieur à 7,9 et inférieur à 8,3.

15. Formulation pharmaceutique selon la revendication 1, dans laquelle le pH de la solution aqueuse est supérieur à 7,9 et inférieur à 8,0.

16. Formulation pharmaceutique selon la revendication 1, comprenant en outre un tampon.

17. Formulation pharmaceutique selon la revendication 16, dans laquelle le tampon est choisi parmi le groupe constitué par le tris(hydroxyméthyl)aminométhane, le phosphate de sodium, la glycylglycine, le citrate et leurs combinaisons.

18. Formulation pharmaceutique selon la revendication 1, dans laquelle le dérivé phénolique est choisi parmi le groupe constitué par le phénol, le m-crésol, le o-crésol, le p-crésol, le chlorocrésol, l'alcool benzylique, le phényl-éthanol, le phénoxyéthanol, le méthylparabène, et leurs combinaisons.

19. Formulation pharmaceutique selon la revendication 18, dans laquelle le dérivé phénolique est choisi parmi le groupe constitué par le phénol, le m-crésol et leurs combinaisons.

20. Formulation pharmaceutique selon la revendication 1, dans laquelle l'agent d'isotonicité est choisi parmi le groupe constitué par le glycérol, le chlorure de sodium, un hydrate de carbone et leurs combinaisons.

21. Formulation pharmaceutique selon la revendication 20, dans laquelle l'hydrate de carbone est choisi parmi le groupe constitué par le saccharose, le dextrose, le mannitol, le tréhalose et leurs combinaisons.

22. Utilisation de la formulation pharmaceutique selon la revendication 1 pour la préparation d'un médicament pour le traitement d'un patient souffrant d'hyperglycémie.

23. Procédé de préparation d'une formulation pharmaceutique appropriée pour l'administration à un patient, comprenant le fait de :

(a) mettre du zinc ou du cobalt à l'état d'oxydation +2 en contact avec un analogue monoacylé de l'insuline humaine ou avec un sel pharmaceutiquement acceptable de l'analogue, dans lequel l'analogue comprend le polypeptide de SEQ ID NO: 1 réticulé de manière appropriée à SEQ ID NO: 2, et dans lequel

Xaa à la position 21 de SEQ ID NO: 1 est choisi parmi le groupe constitué par Asn, Asp, Gly et Glx ;

Xaa à la position 3 de SEQ ID NO: 2 est choisi parmi le groupe constitué par Asn, Asp et Glx ;

Xaa à la position 28 de SEQ ID NO: 2 est choisi parmi le groupe constitué par Asp, Leu, Val, Ala, et Lys acylé;

Xaa à la position 29 de SEQ ID NO: 2 est choisi parmi le groupe constitué par Pro et Lys acylé ; et en outre, dans lequel la position 28 ou la position 29 représente Lys acylé ; et

si la position 28 représente Lys acylé, la position 29 ne représente pas Lys acylé, et

si la position 29 représente Lys acylé, la position 28 ne représente pas Lys acylé ;

(b) régler le pH de la formulation pharmaceutique à une valeur supérieure à environ 7,9 ; et

(c) ajouter un dérivé phénolique et un agent d'isotonicité.

24. Procédé selon la revendication 23, dans lequel Xaa à la position 21 de SEQ ID NO: 1 représente Asn.

25. Procédé selon la revendication 23, dans lequel Xaa à la position 3 de SEQ ID NO: 2 représente Asn.

26. Procédé selon la revendication 23, dans lequel Xaa à la position 21 de SEQ ID NO: 1 représente Asn et dans lequel Xaa à la position 3 de SEQ ID NO: 2 représente Asn.

27. Procédé selon la revendication 23, dans lequel le métal est du zinc.

28. Procédé selon la revendication 23, dans lequel le dérivé phénolique est choisi parmi le groupe constitué par le phénol, le m-crésol, le o-crésol, le p-crésol, le chlorocrésol, l'alcool benzylique, le phényléthanol, le méthylparabène, le résorcinol, le phénoxyéthanol et leurs combinaisons.

29. Procédé selon la revendication 28, dans lequel le dérivé phénolique est choisi parmi le groupe constitué par le phénol, le m-crésol et leurs combinaisons.

30. Procédé selon la revendication 23, comprenant en outre l'addition d'un tampon à la formulation.

31. Procédé selon la revendication 30, dans lequel le tampon est choisi parmi le groupe constitué par le tris(hydroxyméthyl)aminométhane, le phosphate de sodium, la glycylglycine, le citrate et leurs combinaisons.

32. Procédé selon la revendication 23, dans lequel l'agent d'isotonicité est choisi parmi le groupe constitué par le glycérol, le chlorure de sodium, un hydrate de carbone et leurs combinaisons.

33. Procédé selon la revendication 32, dans lequel l'hydrate de carbone est choisi parmi le groupe constitué par le saccharose, le dextrose, le mannitol, le tréhalose et leurs combinaisons.

34. Procédé selon la revendication 23, comprenant en outre le fait de lyophiliser le mélange métal/analogue de l'étape (a).

35. Procédé selon la revendication 23, dans lequel le pH est réglé à une valeur supérieure à 7,9 et inférieure à 8,3.

36. Procédé selon la revendication 23, dans lequel le pH est réglé à une valeur supérieure à 7,9 et inférieure à 8,0.

37. Formulation pharmaceutique appropriée pour l'administration à un patient, comprenant une solution aqueuse dont le pH est supérieur à environ 7,9, la solution aqueuse comprenant :

a) un agent d'isotonicité ;
b) un dérivé phénolique ;
c) des ions de zinc ; et
d) un analogue monoacylé dés(B30) de l'insuline humaine ou un sel pharmaceutiquement acceptable de l'analogue monoacylé dés(B30) de l'insuline humaine.

**38.** Formulation pharmaceutique selon la revendication 37, dans laquelle de 2,5 à 3 moles d'ions de zinc sont présents par 6 moles de l'analogue monoacylé de l'insuline humaine.

**39.** Formulation pharmaceutique selon la revendication 37, dans laquelle de 2 à 3 moles d'ions de zinc sont présents par 6 moles de l'analogue monoacylé de l'insuline humaine.

**40.** Formulation pharmaceutique selon la revendication 37, dans laquelle le pH de la solution aqueuse se situe entre 7,9 et 8,4.

**41.** Formulation pharmaceutique selon la revendication 37, comprenant en outre un tampon.

**42.** Formulation pharmaceutique selon la revendication 41, dans laquelle le tampon est choisi parmi le groupe constitué par le tris(hydroxyméthyl)aminométhane, le phosphate de sodium, la glycylglycine, le citrate et leurs combinaisons.

**43.** Formulation pharmaceutique selon la revendication 37, dans laquelle le dérivé phénolique est choisi parmi le groupe constitué par le phénol, le m-crésol, le o-crésol, le p-crésol, le chlorocrésol, l'alcool benzylique, le phényléthanol, le phénoxyéthanol, le méthylparabène et leurs combinaisons.

**44.** Formulation pharmaceutique selon la revendication 43, dans laquelle le dérivé phénolique est choisi parmi le groupe constitué par le phénol, le m-crésol et leurs combinaisons.

**45.** Formulation pharmaceutique selon la revendication 37, dans laquelle l'agent d'isotonicité est choisi parmi le groupe constitué par le glycérol, le chlorure de sodium, un hydrate de carbone et leurs combinaisons.

**46.** Formulation pharmaceutique selon la revendication 45, dans laquelle l'hydrate de carbone est choisi parmi le groupe constitué par le saccharose, le dextrose, le mannitol, le tréhalose et leurs combinaisons.

**47.** Formulation pharmaceutique selon la revendication 37, dans laquelle l'analogue monoacylé dés(B30) de l'insuline humaine comprend un groupe acyle contenant de 6 à 21 atomes de carbone.

**48.** Formulation pharmaceutique selon la revendication 37, dans laquelle l'analogue monoacylé dés(B30) de l'insuline humaine est choisi parmi le groupe constitué par l'insuline humaine B29-N$^{\epsilon}$-palmitoyl-dés(B30) et par l'insuline humaine B29-N$^{\epsilon}$-myristoyl-dés(B30).

**49.** Formulation pharmaceutique selon la revendication 37, dans laquelle le pH de la formulation est choisi parmi le groupe constitué par 7,97 et 8,4.

**50.** Utilisation de la formulation pharmaceutique selon la revendication 37 pour la préparation d'un médicament pour le traitement d'un patient souffrant d'hyperglycémie.

**51.** Procédé de préparation d'une formulation pharmaceutique appropriée pour l'administration à un patient, comprenant le fait de :

(a) mettre du zinc ou du cobalt à l'état d'oxydation +2 en contact avec un analogue monoacylé dés(B30) de l'insuline humaine ou avec un sel pharmaceutiquement acceptable de l'analogue monoacylé dés(B30) de l'insuline humaine ;
(b) régler le pH de la formulation pharmaceutique à une valeur supérieure à environ 7,9 ; et
(c) ajouter un dérivé phénolique et un agent d'isotonicité.

**52.** Procédé selon la revendication 51, dans lequel le métal est du zinc.

**53.** Procédé selon la revendication 51, dans lequel le dérivé phénolique est choisi parmi le groupe constitué par le phénol, le m-crésol, le o-crésol, le p-crésol, le chlorocrésol, l'alcool benzylique, le phényléthanol, le méthylparabène, le résorcinol, le phénoxyéthanol et leurs combinaisons.

**54.** Procédé selon la revendication 53, dans lequel le dérivé phénolique est choisi parmi le groupe constitué par le phénol, le m-crésol et leurs combinaisons.

**55.** Procédé selon la revendication 51, comprenant en outre l'addition d'un tampon à la formulation.

**56.** Procédé selon la revendication 55, dans lequel le tampon est choisi parmi le groupe constitué par le tris(hydroxyméthyl)aminométhane, le phosphate de sodium, la glycylglycine, le citrate et leurs combinaisons.

**57.** Procédé selon la revendication 51, dans lequel l'agent d'isotonicité est choisi parmi le groupe constitué par le glycérol, le chlorure de sodium, un hydrate de carbone et leurs combinaisons.

**58.** Procédé selon la revendication 57, dans lequel l'hydrate de carbone est choisi parmi le groupe constitué par le saccharose, le dextrose, le mannitol, le tréhalose et leurs combinaisons.

**59.** Procédé selon la revendication 51, dans lequel le pH est réglé à une valeur entre 7,9 et 8,4.

# Figure 1

# Figure 2